# FASCICULE DE BREVET EUROPEEN

(11) **EP 2 364 357 B1**
(45) Date de publication et mention de la délivrance du brevet: **07.05.2014**
(21) Numéro de dépôt: 09760177.7
(22) Date de dépôt: 20.10.2009
(51) Int. Cl.: C12N 1/20, C12R 1/32

(54) **MILIEU ET PROCEDE DE CULTURE DES MYCOBACTERIES INCLUANT LES MYCOBACTERIES DU COMPLEXE MYCOBACTERIUM TUBERCULOSIS**
MYKOBAKTERIEN-KULTURMEDIUM UND VERFAHREN UNTER EINSCHLUSS VON MYKOBAKTERIEN DES MYCOBACTERIUM TUBERCULOSIS-KOMPLEXES
MYCOBACTERIA CULTURE MEDIUM AND METHOD INCLUDING MYCOBACTERIA OF MYCOBACTERIUM TUBERCULOSIS COMPLEX

(30) Priorité: 05.12.2008 FR 0858311
(43) Date de publication de la demande: 14.09.2011
(73) Titulaire: Université d'Aix-Marseille, 13284 Marseille Cedex 07 (FR); Assistance Publique Hôpitaux de Marseille, 13354 Marseille Cedex 5 (FR)
(72) Inventeur: DRANCOURT, Michel, F-13012 Marseille (FR); RAOULT, Didier, F-13008 Marseille (FR)
(74) Mandataire: Domange, Maxime
(86) Numéro de dépôt international: PCT/FR2009/051997
(87) Numéro de publication internationale: WO 2010/063911

(56) Documents cités:
- WHEELER P R ET AL: "Control of acyl-CoA carboxylase activity in mycobacteria" FEMS MICROBIOLOGY LETTERS, BLACKWELL PUBLISHING, AMSTERDAM, NL, vol. 90, no. 2, 1 janvier 1992 (1992-01-01), pages 169-172, XP023921876 ISSN: 0378-1097 [extrait le 1992-01-01]
- REALINI LAURENCE ET AL: "Blood and charcoal added to acidified agar media promote the growth of Mycobacterium genavense" DIAGNOSTIC MICROBIOLOGY AND INFECTIOUS DISEASE, vol. 34, no. 1, mai 1999 (1999-05), pages 45-50, XP002526451 ISSN: 0732-8893

## Description

La présente invention concerne un nouveau milieu de culture des mycobactéries, en particulier des mycobactéries du complexe Mycobacterium tuberculosis, permettant de réduire de façon significative les délais d'isolement par culture et donc le délai de diagnostic des mycobactérioses, en particulier de la tuberculose.

La présente invention concerne également un procédé de culture et d'identification des mycobactéries et, notamment, des bactéries du complexe Mycobacterium tuberculosis.

Plus particulièrement encore, la présente invention concerne un nouveau procédé de décontamination des prélèvements biologiques dans un dit nouveau milieu d'isolement et culture des mycobactéries.

Le présent document concerne également une méthode de détermination phénotypique rapide de la sensibilité d'une mycobactérie, notamment de complexe Mycobacterium tuberculosis, aux antibiotiques, mettant en oeuvre une culture sur un milieu de culture solide selon la présente invention.

Enfin, la présente invention concerne un nouveau procédé d'identification en phase liquide par spectrométrie de masse, contribuant à réduire de façon significative les délais de diagnostic des mycobactérioses, en particulier de la tuberculose.

Les mycobactéries sont des bactéries classées dans le phylum des Actinobacteria par le séquençage du gène 16S ARNr et par les analyses dites "multilocus phylogeny" [Mignard S, Flandrois JP. A seven-gene, multilocus, genus-wide approach to the phylogeny of mycobacteria using supertrees. Int J Syst Evol Microbiol. 2008;58:1432-41] et caractérisées par la présence d'acides mycoliques dans leur paroi, ce qui leur confère une affinité tinctoriale particulière (coloration de Ziehl-Neelsen), et par un chromosome à haut G + C % > 60 % [Pfyffer GE. Mycobacterium : general characteristics, laboratory détection, in staning procédures. In : Murray Pr, Baron EJ, Jorgensen JH, Landry ML, Pfaller MA. Manual of Clinical Microbiology 9ème Eds. Amercian Society for Microbiology, Washington DC ; 2007, pp. 543-572]. Le genre bactérien Mycobacterium comporte plus de soixante espèces, dont des espèces environnementales isolées d'environnements inertes (sol, eau), des espèces associées aux animaux et une espèce strictement humaine, Mycobacterium leprae, agent de la lèpre [Cole S et al. Massive gene decay in the leprosy bacillus. Nature 2001;409:1007-11]. Certaines espèces environnementales sont responsables d'infections opportunistes chez l'homme (les espèces du complexe Mycobacterium avium par exemple) et certaines espèces sont responsables de zoonoses, en particulier certaines espèces de complexe Mycobacterium tuberculosis, responsables de la tuberculose.

Le diagnostic des mycobactérioses repose sur l'isolement et la culture de l'une des espèces du genre Mycobacterium à partir d'un prélèvement clinique réalisé chez l'homme ou chez l'animal. De ce point de vue, le genre Mycobacterium comporte une espèce non-cultivable (Mycobacterium leprae), des espèces de croissance rapide (Mycobacterium fortuitum, Mycobacterium ulcerans, Mycobacterium abscessus) donnant des colonies visibles en moins de sept jours de culture et des espèces à croissance lente donnant des colonies visibles en plus de sept jours de culture, soit en pratique de routine, entre 3 et 8 semaines de culture, notamment avec des milieux Middlebrook. En médecine humaine, les espèces du complexe Mycobacterium avium sont détectables après 10-20 jours de culture et les espèces du complexe Mycobacterium tuberculosis requièrent 10-100 organismes viables par ml de prélèvement et 6-8 semaines de culture pour obtenir 100% de prélèvements positifs [Colebunders R, Bastian I. A review of the diagnosis and treatment of smear-negative pulmonary tuberculosis. Int J Tuberc Lung Dis. 2000;4:97-107]. Etant donné l'importance numérique et la gravité des cas de tuberculose humaine, c'est sur le diagnostic de la tuberculose que les améliorations des techniques de laboratoire sont particulièrement sensibles. En effet, la tuberculose est une maladie infectieuse de l'homme et des animaux due à l'une des sept espèces du complexe Mycobacterium tuberculosis: Mycobacterium tuberculosis, Mycobacterium bovis (et les souches BCG qui en sont dérivées), Mycobacterium africanum, Mycobacterium canettii, Mycobacterium caprae, Mycobacterium microti, et Mycobacterium pinnipedii [Dye C. et al. Prospects for worlwide tuberculosis control under the WHO DOTS strategy. Lancet 1998; 352:1886-91]. L'Organisation Mondiale de la Santé (OMS) a estimé que la tuberculose a été responsable de 9,2 millions de nouveaux cas et de 1,6 millions de décès en 2006 [World Health Organization. 2008. Global tuberculosis control : surveillance, planning, financing. WHO report. Geneva : World Health Organization. WHO/HTM/TB/2008.393]. Ces chiffres soulignent l'importance qu'il y a à optimiser le diagnostic microbiologique de la tuberculose afin d'améliorer la prise en charge médicale des patients et de leur entourage.

L'isolement et la culture des mycobactéries du complexe Mycobacterium tuberculosis sont réalisées à partir de prélèvements cliniques obtenus chez un patient présentant des signes et des symptômes évocateurs de la tuberculose. La forme clinique la plus fréquente, qui est aussi la seule forme clinique contagieuse, est la tuberculose pulmonaire qui est diagnostiquée par l'isolement et la culture d'une mycobactérie du complexe Mycobacterium tuberculosis à partir d'un prélèvement respiratoire tel que l'expectoration, l'aspiration bronchique, le liquide de lavage broncho-alvéolaire obtenu sur bronchoscopie, voire la biopsie pulmonaire. Chez les patients ne produisant pas d'expectoration, le liquide d'aspiration gastrique peut être utilisé comme une alternative pour l'isolement et la culture des mycobactéries du complexe Mycobacterium tuberculosis en cas de tuberculose pulmonaire. Il existe d'autres formes cliniques de tuberculose, en particulier la tuberculose ganglionnaire, mais également les tuberculoses osseuses (Mal de Pott) ainsi que les tuberculoses digestives. En fonction de la forme clinique, différents prélèvements cliniques peuvent être adressés au laboratoire pour isoler et cultiver les mycobactéries du complexe Mycobacterium tuberculosis et diagnostiquer les formes extrapulmonaires.

Pour l'isolement et la culture des mycobactéries du complexe Mycobacterium tuberculosis, on dispose de milieux solides, de milieux liquides, et de milieux biphasiques comportant une phase liquide et une phase solide. Les milieux solides sont fabriqués à base de gélose ou d'agar. Les milieux contenant de l'oeuf entier sont l'utilisation très courante et le milieu le plus utilisé est le milieu de Lowenstein Jensen. Ce milieu, comme les autres milieux contenant de l'oeuf, contient du vert malachite qui participe à inhiber la croissance des microorganismes contaminants. Plusieurs formulations contenant des concentrations variables de vert malachite ont été proposées avec comme résultat constant qu'une diminution de la concentration de vert malachite augmente le ratio de contamination du milieu et une augmentation de la concentration de vert malachite tend à diminuer l'isolement et la culture des mycobactéries du groupe tuberculeux. Une deuxième catégorie de milieux solides sont les milieux à l'agar en particulier le milieu Middlebrook 7H10 et le milieu 7H11 (milieu 7H10 plus 0,1% de caséine hydrolysée). Le milieu de Middlebrook contient 2% de glycérol, qui facilite la culture des mycobactéries du complexe Mycobacterium avium. Les milieux liquides correspondent essentiellement au milieu Middlebrook 7H9.

Cependant, l'isolement des mycobactéries du complexe Mycobacterium tuberculosis est lent puisque la totalité des souches des différentes espèces du complexe Mycobacterium tuberculosis est isolée dans un délai compris entre 6 et 8 semaines. Tout gain de temps sur ce délai représente donc une amélioration significative du diagnostic de laboratoire de la tuberculose et les autres infections à mycobactérie.

Après de nombreuses tentatives, il a été mis au point, selon la présente invention, une formulation nouvelle de milieux de culture permettant l'isolement plus rapide des mycobactéries du complexe Mycobacterium tuberculosis et des autres mycobactéries, notamment permettant une détection et identification en moins de 15 jours, voire même en 10 jours, à partir d'échantillons de prélèvement cliniques. La formulation de milieu de culture selon l'invention permet l'isolement des mycobactéries, aussi bien en milieu liquide adapté aux automates de détection qu'en milieu solide pour une détection manuelle, et, de surcroît, est compatible avec une décontamination à la chlorhexidine dans le cas de prélèvements cliniques pouvant comprendre des bactéries de la flore commensale risquant d'inhiber la croissance des mycobactéries.

Plus précisément, il est fournit un milieu de culture de mycobactéries, comprenant des facteurs de croissance de mycobactéries et, de préférence, des antibiotiques sans activité à l'égard des mycobactéries, caractérisé en ce qu'il comprend les composants additionnels suivants :
- de la lécithine, et
- du sang défibriné, et
- du sérum de veau foetal décomplémenté.

Selon la présente invention, on a donc découvert que l'ajout combiné de sang défibriné, de lécithine et de sérum de veau foetal décomplémenté répondait au but de la présente invention, plus particulièrement dans le cas d'une culture et d'une identification directement à partir d'un bouillon de culture liquide comme il sera explicité ci-après.

Le sérum de veau foetal est un réactif habituellement utilisé pour la culture cellulaire et l'isolement des microorganismes intracellulaires, mais n'est pas un ingrédient utilisé pour l'isolement des bactéries extracellulaires.

Le sérum de veau foetal est décomplémenté, c'est-à-dire que l'on lui a retiré, de façon connue, l'ensemble des protéines appelées "complément sérique" par chauffage, notamment à 56°C pendant une heure. Ce complément sérique est connu pour avoir une activité antibactérienne.

On comprend que le milieu de culture de mycobactéries selon l'invention est constitué d'un milieu de culture de base, connu pour la culture des mycobactéries, comprenant notamment des sels minéraux, sucres, acides aminés, protéines et vitamines, ledit milieu de culture de base étant supplémenté par lesdits composants additionnels mentionnés ci-dessus.

Selon des caractéristiques préférées de réalisation de l'invention :
- la lécithine est comprise dans une proportion pondérale de 0,1 à 5%, de préférence 0,5 à 1%,
- la lécithine est de la lécithine de jaune d'oeuf,
- le sérum de veau foetal décomplémenté est compris dans une proportion en volume de 2,5 à 25%, de préférence de 10 à 20%,
- le sang est compris dans une proportion en volume de 2,5 à 15%, de préférence de 5 à 10%,
- le sang est du sang défibriné de lapin ou, de préférence, de mouton.

Plus particulièrement, un milieu de culture de mycobactéries selon l'invention comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine. Ces composants sont, notamment, les composants contenus dans les milieux de culture de mycobactéries dénommés milieux Middlebrook.

En pratique, ce milieu de culture se présente initialement sous forme d'un liophylisat desdits composants listés ci-dessus, destiné à être dilué dans de l'eau distillée pour former le milieu de culture selon l'invention.

Plus particulièrement encore, lesdits antibiotiques sans activité antimycobactérienne sont les polymyxine, acide nalidixique, triméthoprime, azlocilline et vancomycine, et le milieu de culture comporte, en outre, un antifongique, de préférence l'amphotéricine B.

L'ensemble des antibiotiques et antifungiques cités ci-dessus, à l'exception de la vancomycine, constitue l'ensemble dénommé, connu de l'homme de l'art sous l'appellation PANTA.

Selon une première variante de réalisation de l'invention, ledit milieu de culture est un milieu de culture liquide.

Plus particulièrement, un milieu de culture de mycobactéries selon l'invention comprend un milieu de culture de base qui est un milieu liquide Middlebrook de référence 7H9 et des facteurs de croissance de mycobactéries additionnels suivants : hydrolysat de caséine, glycérol, polysorbate, hemin, acide lactique, biotine, stéarate de polyoxyéthylène, sérum albumine bovine, dextrose et de préférence du supplément H. Le supplément H est une digestion de caséine hautement raffinée.

Selon une seconde variante de réalisation d'un milieu de culture selon l'invention, ledit milieu de culture est un milieu de culture solide contenant un produit gélifiant choisi de préférence parmi les gélose et agar, de préférence dans une proportion pondérale de 0,5 à 5%, de préférence encore de 1 à 2%.

Plus particulièrement, il s'agit d'un milieu aqueux solide de type Middlebrook de référence 7H10, additionné de dit produit gélifiant et des facteurs de croissance de mycobactéries additionnels suivants : acide oléique, albumine bovine, de préférence la fraction V de l'albumine bovine, dextrose, catalase et glycérol. Le vert de malachite, contenu dans le milieu 7H10, est un inhibiteur de croissance des bactéries autres que mycobactéries. Les facteurs de croissance de mycobactéries additionnels ci-dessus, autres que le glycérol, sont connus sous la dénomination facteurs OADC.

La présente invention fournit également un procédé de culture d'une mycobactérie à l'aide d'un milieu de culture de mycobactéries selon l'invention, caractérisé en ce que l'on incube un échantillon contenant une dite mycobactérie, dans un dit milieu de culture de mycobactéries, à une température de 30 à 37°C, appropriée pour la culture de l'espèce de mycobactérie contenue dans l'échantillon.

La plupart des mycobactéries est cultivée à 37°C. Toutefois, certaines mycobactéries, telles que Mycobacterium marinum, Mycobacterium haemophilum, Mycobacterium ulcerans, sont cultivées à une température de 28°C à 30°C. Mycobacterium szulgai peut être cultivée entre 25°C et 37°C, Mycobacterium conspicuum est cultivée entre 22°C et 31°C. La température optimale de croissance de Mycobacterium xenopi et de Mycobacterium shimoidei est de 45°C. Les bactéries Mycobacterium genavense, Mycobacterium kansasii, Mycobacterium fortuitum, les bactéries du complexe Mycobacterium avium, à savoir Mycobacterium avium, Mycobacterium intracellulare, Mycobacterium chimaera et Mycobacterium colombiense sont cultivées à 37°C.

Plus particulièrement, dans un procédé de culture d'une mycobactérie selon l'invention, on cultive un échantillon contenant une bactérie du complexe Mycobacterium tuberculosis à une température de 37°C dans un dit milieu de culture de mycobactéries.

Un procédé de culture de mycobactéries selon l'invention peut également concerner des mycobactéries hors complexe Mycobacterium tuberculosis, telles que celles citées ci-dessus, notamment Mycobacterium marinum, les espèces du complexe Mycobacterium avium, Mycobacterium haemophilum, Mycobacterium xenopi, les espèces du complexe Mycobacterium abscessus, Mycobacterium genavense, Mycobacterium kansasii, Mycobacterium ulcerans et Mycobacterium fortuitum.

On entend ici par "bactéries du complexe Mycobacterium tuberculosis", les bactéries des espèces Mycobacterium tuberculosis, Mycobacterium bovis et ses clones ou sous-espèces BCG, Mycobacterium africanum, Mycobacterium canettii, Mycobacterium caprae, Mycobacterium microti et Mycobacterium pinnipedii.

Avantageusement, dans un procédé de culture d'une mycobactérie selon l'invention, on réalise les quatre étapes suivantes:
- on effectue la culture d'un échantillon de prélèvement biologique pouvant contenir des mycobactéries jusqu'à ce qu'une croissance de bactéries soit détectable, et
- on identifie que la bactérie détectée est une bactérie du genre mycobactérie par un test de coloration, de préférence un test de coloration de Ziehl-Neelsen ou de Kinyoun, et
- le cas échéant, on identifie l'espèce de ladite mycobactérie par des moyens d'analyse moléculaire, de préférence par analyse de la masse moléculaire des protéines bactériennes par spectrométrie de masse.

La coloration de Kinyoun comprend une double coloration avec, successivement, un traitement avec la fushine basique et un traitement avec du bleu de méthylène. La coloration est positive quand il y a coloration bactérienne rouge sur fond cellulaire bleu.

Comme explicité ci-après, les moyens d'analyse moléculaire peuvent être des moyens d'analyse des protéines bactériennes, notamment par détermination de leur masse moléculaire par spectrométrie de masse. Toutefois, alternativement, on pourra avoir recours aux méthodes classiques d'identification moléculaire du génome (ADN ou ARN) avec des sondes et/ou amorces d'amplification spécifiques des différentes espèces de mycobactéries, notamment telles que décrites dans la demande WO 2008/050064. En particulier, pour la détection de mycobactéries du complexe Mycobacterium tuberculosis, on peut avoir recours à un système appelé multi spacer sequence typing (abrégé ci-après en MST) constitué d'une série de fragments d'acides nucléiques de zones intergéniques non codantes du génome des bactéries du complexe Mycobacterium tuberculosis, lesdits fragments constituant des marqueurs génétiques permettant l'identification des différentes espèces du complexe Mycobacterium tuberculosis et le génotypage des isolats d'une même espèce du complexe Mycobacterium tuberculosis et, notamment de l'espèce Mycobacterium tuberculosis, par l'analyse des séquences desdits fragments de zones appelés MST.

Avantageusement, on détecte une croissance de bactérie du complexe Mycobacterium tuberculosis en moins de 15 jours, de préférence en pas plus de 10 jours.

Les milieux de culture selon l'invention permettent en outre la détection de 50% de l'ensemble des différentes principales espèces de mycobactéries en moins d'une semaine, à savoir Mycobacterium marinum, Mycobacterium avium, les bactéries du complexe Mycobacterium tuberculosis, à savoir les bactéries Mycobacterium tuberculosis, Mycobacterium bovis, Mycobacterium africanum, Mycobacterium canettii, Mycobacterium caprae, Mycobacterium pinnipedii et Mycobacterium microti.

Dans un mode préféré de réalisation dans lequel on réalise une culture d'un échantillon de prélèvement biologique pouvant contenir une mycobactérie et des bactéries contaminantes dont la croissance peut inhiber la croissance des mycobactéries, le procédé est caractérisé en ce que :
1/ on réalise une étape préalable de décontamination initiale dudit échantillon dans ledit milieu de culture avec de la chlorhexidine pendant une période limitée, afin de limiter l'action de la chlorhexidine à une activité contre les bactéries autres que mycobactéries, de préférence environ 15 minutes de traitement dans une solution de chlorhexidine à 1% sous agitation, et
2/ on élimine la chlorhexidine en lavant l'échantillon ainsi traité de l'étape 1/ avec un tampon neutre, et on centrifuge et récupère le culot bactérien contenant les bactéries contaminantes ainsi inactivées et les mycobactéries non inactivées, que l'on inocule sur un dit milieu de culture de mycobactéries.

L'utilisation de chlorhexidine comme décontaminant était limitée, précédemment, à la décontamination d'échantillons dans des milieux de culture solides, car la chlorhexidine entraîne une précipitation des milieux de culture liquides. En outre, la chlorhexidine est, en principe, connue pour être toxique sur les mycobactéries. Toutefois, on a découvert selon la présente invention que la présence de lécithine dans le milieu de culture permettait d'éviter ou de différer la précipitation du milieu de culture liquide, d'une part, et, d'autre part, l'action limitée dans le temps de la chlorhexidine, par la mise en oeuvre de ladite étape de lavage 2/, permet d'inhiber l'activité postérieure de la chlorhexidine à l'égard des mycobactéries. La chlorhexidine conserve toutefois son activité première contre les bactéries de la flore commensale dont la croissance peut inhiber la croissance des mycobactéries, appelées ci-dessus bactéries contaminantes.

On procède, en particulier, à cette étape de décontamination préalable pour les prélèvements biologiques connus pour être porteurs d'une flore commensale, tels que des prélèvements d'expectoration, de biopsie cutanée ou de selles, lesquels représentent 95% des prélèvements biologiques disponibles pour une analyse de mycobactéries. En revanche, pour des prélèvements biologiques tels que provenant d'hémoculture ou de biopsie ganglionnaire ou de biopsie pulmonaire ou de biopsie osseuse, une telle décontamination n'est pas requise.

Le procédé ci-dessus est donc particulièrement avantageux lorsque ledit milieu de culture de mycobactéries est un milieu de culture liquide de mycobactéries.

Le procédé ci-dessus est aussi particulièrement avantageux lorsque ledit prélèvement biologique est effectué à partir de selles.

Dans une variante de réalisation, ledit milieu de culture est un dit milieu de culture liquide de mycobactéries et on détecte la croissance de dites mycobactéries par analyse périodique de la concentration en oxygène au sein de l'enceinte de culture.

De préférence, la mesure de la concentration en oxygène est effectuée toutes les au plus 3 heures jusqu'à ce qu'un seuil de concentration en oxygène soit atteint, correspondant de préférence à une croissance de bactéries correspondant à une concentration de mycobactéries d'au moins 10⁴ bactéries/ml.

Cette méthode de détection de bactéries par mesure de la concentration en oxygène est connue et des automates de détection sont commercialisés. Selon les automates de détection, la concentration minimale en mycobactéries, à partie de laquelle on détecte une consommation significative d'oxygène et donc une baisse significative de la concentration en oxygène, varie et correspondant, en général, à des concentrations de 10³ à 10⁴ mycobactéries/ml.

Dans une autre variante de réalisation, ledit milieu de culture est un milieu de culture solide et on détecte une croissance de dites mycobactéries à l'oeil nu lorsque l'on peut apercevoir la formation d'une colonie de bactéries sur ledit milieu de culture solide.

En général, l'observation de la formation d'une colonie bactérienne correspondant à une concentration en bactéries de 10⁶ bactéries/ml.

Dans un mode préféré de réalisation d'un procédé selon l'invention, l'on identifie l'espèce de bactéries du genre mycobactérie par des moyens d'analyse moléculaire consistant dans une analyse du profil protéique obtenu par spectrométrie de masse, en le comparant à une série de spectres de profil protéique obtenus avec des échantillons de souche de mycobactéries de référence de différentes espèces cultivées dans les mêmes conditions de culture.

On entend ici par "mêmes conditions de culture" que l'on a recours au même milieu de culture, même température de culture pour une même espèce de mycobactérie.

La présente invention fournit également un procédé de culture et d'identification d'une mycobactérie dans un prélèvement d'échantillon biologique, caractérisé en ce que l'on effectue la culture dudit échantillon de prélèvement biologique dans un dit milieu de culture de mycobactéries liquide, et, pour la réalisation de l'analyse de spectrométrie de masse, on analyse un culot bactérien obtenu par double centrifugation, directement à partir du bouillon de culture selon les étapes suivantes :
a- On inactive les mycobactéries mises en suspension d'un échantillon de prélèvement biologique cultivé, dans lequel on a détecté la croissance de bactéries, par chauffage à plus de 90°, de préférence à 95°C pendant une heure, de préférence sous agitation, et
b -on réalise une première centrifugation à basse vitesse, de préférence à 500 tr/mn, du surnageant d'un dit échantillon de prélèvement biologique cultivé, dans lequel on a détecté la croissance de bactéries, cette première centrifugation étant réalisée jusqu'à sédimenter les globules rouges du sang contenu dans ledit échantillon, et
c- on récupère le surnageant de la première centrifugation de l'étape a-, et on réalise une deuxième centrifugation à grande vitesse, de préférence au moins 10 000 tr/mn, de préférence environ 14 000 tr/mn, cette deuxième centrifugation étant réalisée jusqu'à obtenir sédimentation d'un culot bactérien, et
d- de préférence, on lave ledit culot bactérien de l'étape b- avec un tampon neutre, tel que du PBS, et on réalise un traitement chimique, de préférence avec un mélange d'acétonitrile et d'acide trifluoroacétique, pour séparer les protéines de la bactérie et les rendre analysable par spectrométrie de masse, et
e- on récupère le culot protéique bactérien de l'étape d- que l'on dépose sur une plaque de spectrométrie de masse.

La méthode d'inactivation de l'étape a- s'est révélée compatible avec l'obtention de spectres de masse de bonne qualité à l'étape e-.

Cette méthode d'identification directement à partir d'un échantillon de milieux liquides par spectrométrie de masse est particulièrement avantageuse de par sa rapidité, dans la mesure où, dans la technique antérieure, on analysait par spectrométrie de masse uniquement des souches bactériennes prélevées directement à partir d'un milieu solide. Ainsi, dans le cas de prélèvement clinique cultivé dans un milieu liquide, les bactéries étaient ré inoculées puis cultivées sur un milieu solide avant d'être prélevées pour identification par spectrométrie de masse, ce qui présentait une durée additionnelle de 3 à 8 semaines, selon le type de mycobactéries, en particulier de 6 à 8 semaines de culture sur ledit milieu solide pour les bactéries du complexe Mycobacterium tuberculosis.

Selon la présente invention, à partir d'un culot bactérien obtenu directement du milieu liquide de culture, on peut réaliser directement l'analyse de spectrométrie de masse.

Au total, en comptabilisant le temps des étapes de chauffage et des étapes de centrifugation a- à e- ci-dessus, il est possible de réaliser le spectre d'analyse protéique par spectrométrie de masse environ 1h30 après la détection de croissance desdites bactéries par culture, laquelle, comme rappelé précédemment dans le cas des mycobactéries du complexe Mycobacterium tuberculosis, peut être obtenue pas plus d'environ 15 jours, voire 10 jours.

Le présent document concerne également une méthode de détermination phénotypique rapide de la sensibilité des mycobactéries, notamment de Mycobacterium tuberculosis, aux antibiotiques.

Le traitement de la tuberculose repose sur une polychimiothérapie combinant cinq antibiotiques, rifampicine, isoniazide, ethambutol et pyrazinamide qui sont appelés avec la streptomycine, antituberculeux de première ligne. On constate dans le monde entier l'émergence de souches de M. tuberculosis dites « multi-drug resistant » (MDR-TB) résistantes à la rifampicine et à l'isoniazide et résistantes et de souches extensivement résistantes (XDR-TB) qui sont des souches MDR-TB également résistantes aux antituberculeux de deuxième ligne (fluoroquinolones, aminosides, capréomycine) [Gagneux S. Clin Infect Dis. 2009 ; 15 : S1 :66-68]. En France, leur prévalence est estimée à 15%. Le terme de résistance primaire concerne une souche résistante isolée chez un patient qui n'a jamais reçu de traitements antituberculeux, le terme de résistance secondaire concerne la souche résistante isolée chez un patient qui a bénéficié d'un traitement antituberculeux antérieurement à l'isolement de la souche [Schluger NW, Up-to-date 2009].

Les souches résistantes du complexe Mycobacterium tuberculosis posent un problème thérapeutique car elles imposent l'administration d'antibiotiques antituberculeux de deuxième ligne qui ont pour les inconvénients suivants :
(1) d'être moins efficaces que les antibiotiques antituberculeux de première ligne,
(2) de devoir être administrés par voie parentérale contrairement aux antibiotiques antituberculeux de première ligne,
(3) d'être plus toxiques que les antibiotiques antituberculeux de première ligne.

Le pronostic de la tuberculose étant lié à l'administration d'antibiotiques efficaces, il est indispensable de déterminer dans les délais les plus brefs la sensibilité des souches aux antituberculeux (antibiogramme). Les méthodes génotypiques détectant la présence de mutations dans les gènes-cibles, repose sur une technologie et une expertise qui n'est pas largement diffusée [Woods GL, Warren NG, Inderlind CB. Susceptibility test methods : Mycobacteria, Nocardia, and over actinomycetes in: Annuel of Clinical Microbiology, 9ème édition (Nurray PR, Baren EJ, Jorgensen JH, Bry NL, Psaller MA. American Society for Microbiology, 2007, page 1223-1247]. Pour les antibiotiques autres que la rifampicine, l'association statistique entre mutations du gène cible et phénotype de résistance est faible. C'est pourquoi, les tests phénotypiques gardent tout leur intérêt. Ceux-ci peuvent être automatisés en milieu liquide ; mais cette technique ne permet de tester qu'une ou deux concentrations d'antibiotique par souche et tolère la confusion entre souche de M. tuberculosis résistante et contaminant [Anthony RM et al. Int J Tuberc Lung Dis. 2009 ; 13 :1051-1053]. Il est donc préférable de réaliser les tests en milieu solide, permettant la discrimination des colonies de contaminant. Ces tests reposent sur l'observation de l'inhibition de croissance du complexe M. tuberculosis en présence d'une concentration connue de l'antibiotique, par rapport à un témoin de croissance sans antibiotique. Les limites actuelles des tests phénotypiques en milieu solide sont le délai de 3-4 semaines pour l'obtention du résultat et la manipulation de grande quantité de M. tuberculosis (pathogène de classe 3) correspondant à un milieu par concentration d'antibiotique testé.

Un milieu de culture solide selon la présente invention permet de réduire le délai de croissance des colonies de M. tuberculosis en présence d'antibiotique et donc obtenir rapidement des résultats d'antibiogramme. Par ailleurs, on a découvert selon la présente invention qu'il était possible d'utiliser avantageusement le procédé E-test pour la réalisation des antibiogrammes de M. tuberculosis. Le E-test (AB Biodisk, Solna, Suède ; BioMérieux, Marcy-l'Etoile, France) consiste en une bande de papier imprégné d'un gradient de concentration d'un antibiotique étudié, permettant une lecture directe de la concentration provoquant l'inhibition de croissance de la bactérie (Concentration Minimale Inhibitrice, CMI). Le E-test a été validé pour M. tuberculosis sur des milieux de référence différents de celui selon l'invention [Esteban J. et al. Eur J Clin Microbiol Infect Dis 2005 ; 24: 856-857] et les caractéristiques du milieu de culture solide selon l'invention ne permettaient pas prévoir s'il serait approprié à la réalisation des E-tests.

On a démontré selon la présente invention que le milieu de culture solide selon l'invention permet également une réalisation plus rapide et une lecture plus facile d'un test phénotypique de sensibilité et résistance aux antibiotiques du complexe Mycobacterium tuberculosis que les milieux standards.

La présente invention permet donc de mettre à profit l'invention d'un milieu permettant l'isolement et la croissance rapide du complexe Mycobacterium tuberculosis, pour la réalisation rapide de tests phénotypiques de sensibilité du complexe Mycobacterium tuberculosis aux antibiotiques antituberculeux comme illustré dans l'exemple.

Plus précisément, le présent document fournit un procédé de culture permettant la détermination phénotypique rapide de la sensibilité d'une mycobactérie aux antibiotiques, selon laquelle on réalise les étapes dans lesquelles :
i/- on réalise une culture d'une dite mycobactérie, de préférence du complexe Mycobacterium tuberculosis, sur dit un milieu de culture solide en présence d'au moins un antibiotique donné, à différentes concentrations connues, et
ii/- on détermine la plus petite concentration d'antibiotique, de préférence choisi parmi la rifampicine, isoniazide, éthambutol, pyrazinamide et la streptomycine, qui inhibe toute croissance visible de ladite bactérie (CMI : concentration minimale inhibitrice).

Ainsi, de façon connue, pour chaque couple de mycobactérie-antibiotique, on peut déterminer une concentration minimale inhibitrice ou CMI et la comparer aux concentrations critiques d'antibiotiques qu'un malade peut recevoir sans danger et qui bloque la croissance de la souche bactérienne en cause. On détermine alors la sensibilité ou la résistance de la bactérie à l'antibiotique comme suit :
- si la CMI est inférieure à la concentration critique inférieure, la bactérie est sensible à l'antibiotique, et
- si la CMI est supérieure à la concentration critique supérieure, la bactérie est résistante à l'antibiotique en cause.

Dans un mode préféré de réalisation selon l'invention, à l'étape i/- , on dépose, sur ledit milieu de culture solide, au moins une bandelette de papier imprégné d'un dit antibiotique, à différentes concentrations selon un gradient de concentration le long de ladite bandelette.

Plus particulièrement, on ensemence une boite de Pétri comprenant ledit milieu de culture solide selon l'invention à une concentration d'au moins 10⁴ colonies/ml, de préférence 10⁶ colonies/ml, puis on dépose une dite bandelette sur ledit milieu de culture solide, notamment une bandelette de type E-test qui comprend un gradient continu en concentrations croissantes d'antibiotique d'une extrémité à l'autre de ladite bandelette, lesdites concentrations d'antibiotique étant écrites directement sur la bandelette, et on met en incubation la boite sur laquelle repose la bandelette et on lit la CMI à l'intersection du disque d'inhibition de la croissance bactérienne avec la bandelette.

Selon d'autres modes de réalisation connus, on peut remplacer la bandelette par un disque de papier buvard imprégné de la concentration critique d'antibiotique, afin de vérifier l'existence d'une zone d'inhibition autour du disque pour établir que la souche est sensible à l'antibiotique.

Selon une autre variante de réalisation connue, on peut incorporer l'antibiotique directement dans la masse dudit milieu de culture solide à ladite concentration critique et comparer la croissance de la souche sur ledit milieu de culture solide imprégné dudit antibiotique, avec la croissance d'une même souche sur un même milieu de culture solide ne contenant pas d'antibiotique, pour vérifier la présence ou l'absence d'une croissance bactérienne dans le milieu de culture avec et, respectivement, sans antibiotique et en déduire que la souche est sensible à l'antibiotique si l'on observe une croissance moindre de la souche dans le milieu de culture solide contenant ledit antibiotique.

D'autres caractéristiques et avantages de la présente invention ressortiront mieux à la lecture des exemples qui vont suivre, faits en référence aux figures 1 à 7, dans lesquels :
les figures 1 à 4 représentent des graphes représentant le délai cumulatif T (en heure) pour la détection d'un nombre de bactéries, indiqué de 1 jusqu'à 10, de 10 souches de Mycobacterium tuberculosis pour un milieu de culture de référence (figure 1) et des milieux de culture selon l'invention (figures 2 à 4);
la figure 5 représente une photographie de deux colonies de mycobactéries cultivées sur milieu solide selon l'exemple 2;
La figure 6 représente une photographie de coloration de Ziehl-Neelsen d'une colonie cultivée sur milieu solide à l'exemple 2;
Les figures 7A à 7B représentent des spectres obtenus par spectrométrie de masse à l'exemple 5;
- les figures 8A et 8B représentent des photographies d'une croissance bactérienne sur un milieu de culture solide de référence (figure 8A) et un milieu de culture solide selon l'invention (figure 8B) en contact avec des bandelettes de papier buvard imprégnées d'antibiotique.

### EXEMPLE 1 : CULTURE DES MYCOBACTERIES DU GROUPE TUBERCULEUX EN MILIEU LIQUIDE

Dans cet exemple, les inventeurs ont inoculé des souches de Mycobacterium tuberculosis en parallèle dans le milieu Middelbrook 7H9 de référence avec suppléments F et PANTA (Becton-Dickinson, Grenoble, France) et dans un des trois milieux de leur invention dont la composition est donnée ci-dessous pour un flacon de 40ml dans lequel volume les tests ont été réalisés:

### Exemple 1A : Composition finale du milieu B25% (pour 1000 ml)

### 1- Milieu de Middlebrouk 7H9

Eau distillée stérile: 750 ml
Sulfate d'ammonium : 1 g
Acide L-glutamique : 0,5 g
Citrate de sodium : 0,1 g
Pyridoxine : 1 mg
Biotine : 0,5 mg
Phosphate disodique : 2,5 g
Phosphate monopotassique : 1 g
Citrate d'ammonium ferrique : 0,1 g
Sulfate de magnésium : 0,05 g
Chlorure de calcium : 0,5 mg
Sulfate de zinc : 1 mg
Sulfate de cuivre : 1 mg
Hydrolysat de caséine : 1 g
Supplement H: 3 g
Glycérol: 1 g
Polysorbate 80 : 0,05 g
Hemin : 0,005 g

### 2- Facteurs de croissance additionnels :

Acide lactique : 168 mg
Stéarate de polyoxyéthylène : 110 mg
Sérum albumine bovine : 5,6 g
Dextrose : 1100 mg
Biotine: 0,57 mg

### 3- Antibiotiques sans activités antimycobactérienne et antifungique :

Polymyxine : 40.000 unités
Acide nalidixique : 16 mg
Triméthoprime : 4 mg
Azlocilline : 4 mg
Vancomycine : 20 mg
Amphothéricine B : 4 mg

### 4- Eléments additionnels de l'invention :

Sérum de veau foetal décomplémenté: 250 ml
Sang de mouton défibriné : 50 ml
Lécithine : 0,5 g

### Exemple 1B : Composition finale du milieu C15% (pour 1000 ml)

### 1- Milieu de Middlebrook 7H9

Eau distillée stérile: 750 ml
Sulfate d'ammonium : 1 g
Acide L-glutamique : 0,5 g
Citrate de sodium : 0,1 g
Pyridoxine : 1 mg
Biotine : 0,5 mg
Phosphate disodique : 2,5 g
Phosphate monopotassique : 1 g
Citrate d'ammonium ferrique : 0,1 g
Sulfate de magnésium : 0,05 g
Chlorure de calcium : 0,5 mg
Sulfate de zinc : 1 mg
Sulfate de cuivre : 1 mg
Hydrolysat de caséine : 1 g
Supplement H: 3 g
Glycérol: 1 g
Polysorbate 80 : 0,05 g
Hemin : 0,005 g

### 2-Facteurs de croissance additionnels :

Acide lactique : 168 mg
Stéarate de polyoxyéthylène : 110 mg
Sérum albumine bovine : 5,6 g
Dextrose : 1100 mg
Biotine: 0,57 mg

### 3- Antibiotiques sans activité antimycobactérienne et antifungique

Polymyxine : 40.000 unités
Acide nalidixique : 16 mg
Triméthoprime : 4 mg
Azlocilline : 4 mg
Vancomycine : 20 mg
Amphothéricine B : 4 mg

### 4- Eléments additionnels de l'invention :

Sérum de veau foetal décomplémenté: 150 ml
Sang de mouton défibriné : 50 ml
Lécithine : 0,5 g

### Exemple 1C : Composition finale du milieu D5% (pour 1000 m)

### 1- Milieu de Middlebrook 7H9 :

Eau distillée stérile: 750 ml
Sulfate d'ammonium : 1 g
Acide L-glutamique : 0,5 g
Citrate de sodium : 0,1 g
Pyridoxine : 1 mg
Biotine : 0,5 mg
Phosphate disodique : 2,5 g
Phosphate monopotassique : 1 g
Citrate d'ammonium ferrique : 0,1 g
Sulfate de magnésium : 0,05 g
Chlorure de calcium : 0,5 mg
Sulfate de zinc : 1 mg
Sulfate de cuivre : 1 mg
Hydrolysat de caséine : 1 g
Supplement H: 3 g
Glycérol: 1 g
Polysorbate 80 : 0,05 g
Hemin : 0,005 g

### 2- Facteurs de croissance additionnels :

Acide lactique : 168 mg
Stéarate de polyoxyéthylène : 110 mg
Sérum albumine bovine : 5,6 g
Dextrose : 1100 mg
Biotine: 0,57 mg

### 3- Antibiotiques sans activité antimycobactérienne et antifungique:

Polymyxine : 40.000 unités
Acide nalidixique : 16 mg
Triméthoprime : 4 mg
Azlocilline : 4 mg
Vancomycine : 20 mg
Amphothéricine B : 4 mg

### 4- Eléments additionnels de l'invention :

Sérum de veau foetal décomplémenté: 50 ml
Sang de mouton défibriné : 50 ml
Lécithine : 0,5 g

Dans cet exemple, dix souches cliniques de Mycobacterium tuberculosis ont été inoculées en parallèle dans un milieu A0% témoin et dans les trois milieux de l'invention notés B25%, C15% et D5%. Ces dix souches comportaient six souches sensibles aux antibiotiques antituberculeux, une souche Mycobacterium tuberculosis n°9 résistante à la streptomycine et à l'izoniazide, une souche Mycobacterium tuberculosis n°10 résistante à la streptomycine et à la rifadine et deux souches Mycobacterium tuberculosis n°11 et n°12 résistantes à la streptomycine, à la rifampicine, à l'éthambutol et à l'izoniazide (souches multirésistantes dites multidrug-resistant MDR). Pour une même souche, le même inoculum a été ensemencé en parallèle dans les quatre milieux. Les milieux liquides ont été préparés dans des flacons en verre incubés dans un automate BACTEC 9000MB (Becton-Dickinson, Grenoble, France) à température constante de 37°C. L'expérimentation a été reproduite trois fois avec les mêmes souches. L'automate détecte dans les flacons la présence d'une consommation d'oxygène, témoin d'une croissance bactérienne, et indique le délai entre cette détection et l'introduction du flacon dans l'automate. Ce délai est le critère utilisé dans cet exemple. Le critère de jugement dans cet exemple est donc le délai de détection par l'automate BACTEC 9000MB d'une croissance de mycobactéries identifiées.

Dans cet exemple, la spécificité de la détection de mycobactéries par l'automate a été affirmée après coloration de Gram sur le surnageant du flacon (pour vérifier l'absence de bactérie contaminante), après coloration de Kinyoun sur le surnageant du flacon (pour vérifier la présence de bacilles alcoolo-acido-résistants, caractéristiques des mycobactéries) puis par amplification PCR en temps réel de la séquence d'insertion IS6110 spécifique des bactéries du complexe Mycobacterium tuberculosis.

La coloration de Kinyoun a été réalisée selon le protocole suivant : Après réalisation d'une lame de cytospin, la lame est mise en immersion dans du méthanol pendant 10 min, puis en immersion dans une solution de Kinyoun (Fushine basique40 g, phénol 80 ml, alcool éthylique 200 ml, eau pour un volume final de un litre) pendant trois heures. Après rinçage à l'eau, la lame est recouverte d'une solution de Gabett (Bleu de méthylène 10 g, acide sulfurique 60°B 200 ml, acool absolu 300 ml et eau pour un volume final de 500 ml) pendant cinq minutes, rincée à l'eau, séchée et lue au microscope optique à grossissement X 1.000. La coloration est positive quand il y a une coloration bactérienne rouge sur fond cellulaire bleu.

La séquence d'insertion IS6110, spécifique des bactéries du complexe Mycobacterium tuberculosis est décrite dans [van Embden JD and coll. Strain identification of Mycobactrium tuberculosis by DNA Fingerprinting : recommendation for a standardized methodology. J. Clin. Microbiol. 1993 ;31 :406-409].

Les inventeurs ont également observé que l'ajout de 5 %, 15% ou 25% de sang de mouton défibriné n'induisait pas de consommation d'O2 détectée par l'automate et donc pas de faux positif; cette expérience a été réalisée trois fois.

Les inventeurs ont ainsi testé des souches types des plusieurs espèces du genre Mycobacterium provenant de collections publiques ainsi que quelques souches cliniques issues de l'activité diagnostique de leur laboratoire y compris une souche de M. marinum.

Les souches types étaient : M. tuberculosis H37RV, M. bovis CIP 105050, M. bovis BCG vaccine CIP 105060, M. africanum CIP 105147^{T}, M. pinnipedii CIP 7177, M. avium ss para tuberculosis ATCC 19698^{T}.

Le délai de détection est toujours écourté dans le cas de culture avec les milieux de culture selon l'invention en comparaison avec un milieu Middlebrook 7H9, notamment de 375 à 55 heures pour Mycobacterium africanus, de 336 à 29 heures pour Mycobacterium pinnipedii et de 450 à 35 heures pour Mycobacterium bovis.

Pour l'espèce Mycobacterium tuberculosis, les inventeurs ont pris soin de tester des souches cliniques sensibles aux antituberculeux et des souches cliniques résistantes à certains antituberculeux.

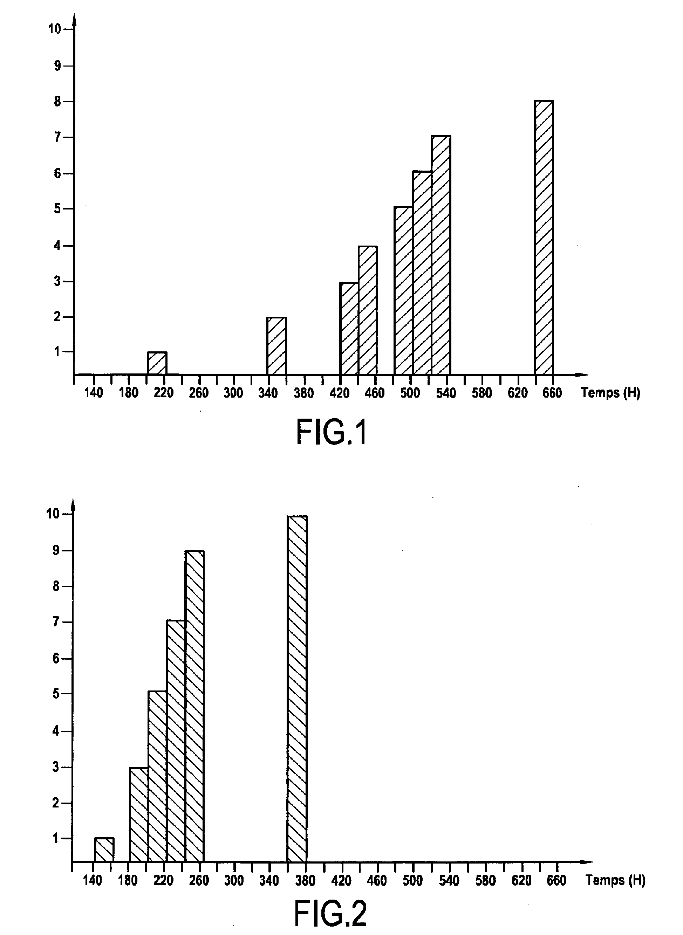

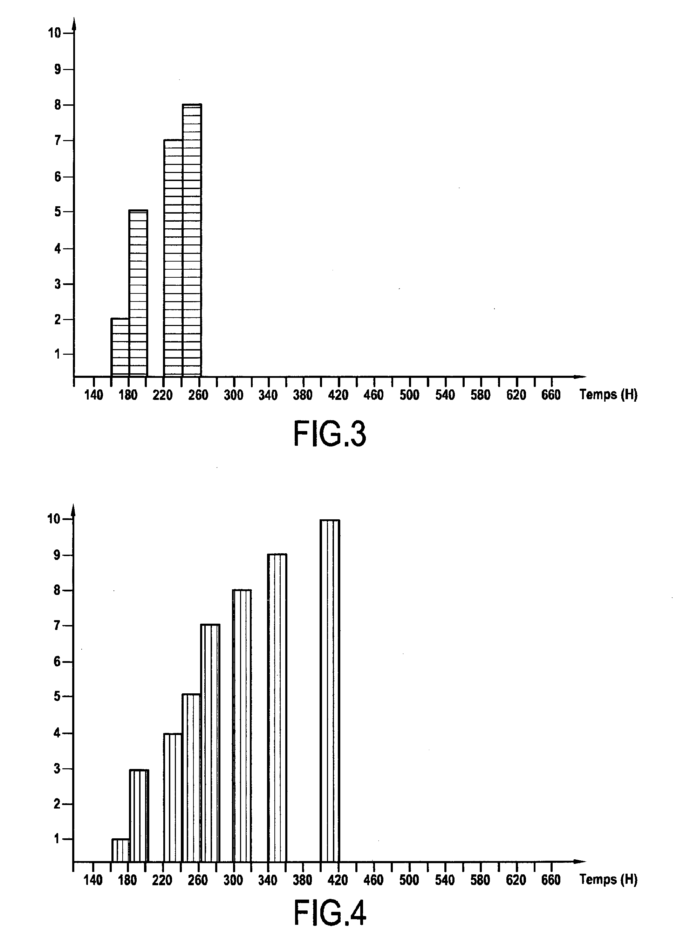

Des résultats de ces tests sont donnés dans les graphes des figures 1 à 4, où sont représentés, en ordonnées, un nombre de souches d'espèces différentes du complexe Mycobacterium tuberculosis et, en abscisses, le délai cumulatif exprimé en heures (t) pour la détection du nombre de souches indiqué en ordonnées allant jusqu'à dix souches d'espèces différentes du complexe Mycobacterium tuberculosis pour chacun des trois milieux de culture de l'invention (milieux B25% (figure 2), C15% (figure 3) et D5% (figure 4)) par rapport au milieu A0% de référence (figure 1).

Ces graphes illustrent :
1/- la supériorité des milieux de culture de l'invention pour la culture de Mycobacterium tuberculosis puisque le temps de culture pour observer une détection de croissance bactérienne est inférieur dans les milieux de l'invention par rapport au milieu de référence,
2/- le fait que les milieux de culture de l'invention permettent la culture des souches de Mycobacterium tuberculosis indépendamment de leur profil de résistance aux antibiotiques antituberculeux.

La détection d'une croissance bactérienne était toujours possible en moins de 15 jours, voire moins de 10 jours.

En outre, le délai médian permettant la détection de 50% des souches inoculées est inférieur ou égal à une semaine, avec les milieux de culture selon l'invention.

### EXEMPLE 2 : CULTURE DES MYCOBACTERIES EN MILIEU SOLIDE

Dans cet exemple, les inventeurs ont inoculé en parallèle différents milieux solides contenant de la gélose avec trois souches cliniques de Mycobacterium tuberculosis. Les milieux étaient (1) Middlebrook 7H10 plus gélose 0,5% (2) Middlebrook 7H10 plus gélose 1% plus 5% de sang défibriné de mouton plus 5% de sérum de veau foetal plus 0,5% de lécithine d'oeuf (3) Middlebrook 7H10 plus gélose 0,5% plus 5% de sang défibriné de mouton plus 15% de sérum de veau foetal plus 0,5% de lécithine d'oeuf (4) Middlebrook 7H10 plus gélose 0,5% plus 5% de sang défibriné de mouton plus 25% de sérum de veau foetal plus 0,5% de lécithine d'oeuf. Deux géloses témoins ont été systématiquement utilisées en parallèle, une gélose Middlebrook 7H10 plus gélose 1% et une gélose Middlebrook 7H10 plus gélose 1% plus 5% de sang défibriné de mouton plus 5% de sérum de veau foetal plus 0,5% de lécithine d'oeuf. Les géloses témoins (non-inoculées) et les géloses inoculées ont été incubées à 37°C sous une atmosphère contenant 5% de CO₂ dans des sachets en plastique empêchant la déshydratation. L'inspection des géloses était quotidienne.

Le critère de jugement utilisé dans cet exemple est le délai d'apparition de colonies détectées par inspection à l'oeil nu des géloses et confirmées comme étant des colonies de Mycobacterium tuberculosis par coloration de Ziehl-Neelsen et séquençage.

Les milieux solides utilisés ont la composition suivante :

Pour confirmer avec certitude l'apparition de colonies, les inventeurs ont photographié les dites colonies. Les inventeurs ont ensuite réalisé une coloration de Kinyoun à partir d'un prélèvement de ladite colonie, et à distance, à partir d'une région de la même gélose ne présentant pas de colonie. Dans tous les cas, il n'a pas été détecté de bacilles alcoolo-acide-résistants à distance de la colonie, alors qu'il a été détecté de tels bacilles dans les colonies.

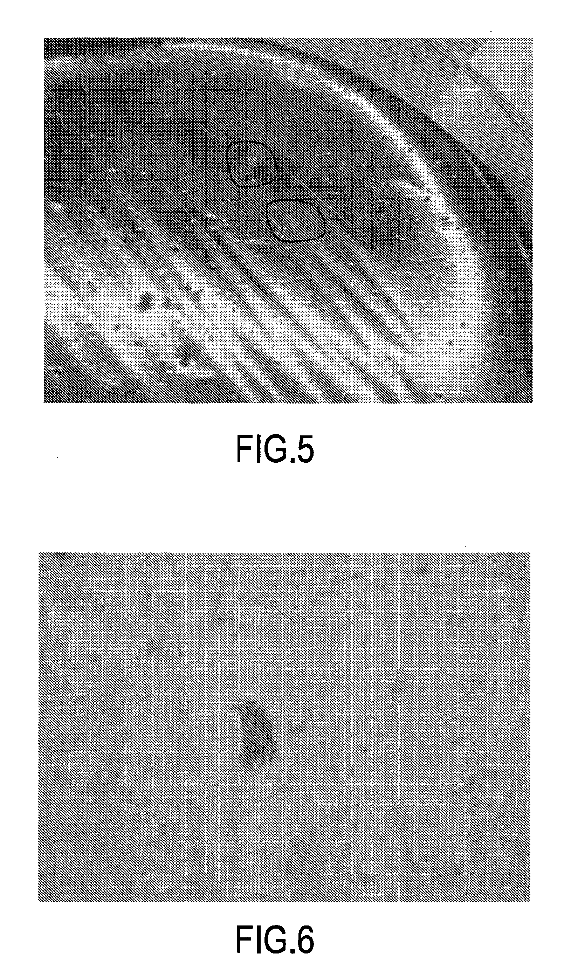

Un exemple de colonies est présenté dans la figure 6.

La figure 5 représente une gélose contenant le milieu C15% inoculé depuis 10 jours avec une souche clinique Mycobacterium tuberculosis, montrant deux colonies (cercles).

La figure 6 représente la coloration de Ziehl-Neelsen pratiquée à partir d'une colonie obtenue en 10 jours à partir d'une souche clinique de Mycobacterium tuberculosis sur le milieu solide C15%.

### Exemple 2A : Milieu solide B25% pour 1000 ml:

### 1- Milieu de Middlebrook 7H10 :

Eau distillée stérile: 750 ml
Sulfate d'ammonium : 0,50 g
Phosphate monopotassique : 1,50 mg
Phosphate disodique : 1,50 g
Citrate de sodium : 0,4 g
Sulfate de magnésium : 0,025 g
Chlorure de calcium : 0,00050 g
Sulfate de zinc : 0,0010 g
Sulfate de cuivre : 0,0010 g
Acide L-glutamique : 0,50 g
Citrate d'ammonium ferrique : 0,04 g
Chlorhydrate de pyridoxine : 0,0010 g
Biotine : 0,000050 g
Vert malachite : 0,0000250 g
Gélose: 15 g

### 2- Facteurs de croissance additionnels:

Chlorure de sodium : 8,50 g
Albumine bovine (fraction V) : 50 g
Dextrose : 20 g
Catalase : 0,030 g
Acide oléique : 0,60 ml
Glycérol: 0,5%

### 3- Antibiotiques sans activité antimycobactérienne et antifungique :

Polymyxine : 40.000 unités
Amphothéricine B : 4 mg
Acide nalidixique : 16 mg
Triméthoprime : 4 mg
Azlocilline : 4 mg
Vancomycine : 20 mg

### 4- Eléments additionnels de l'invention :

Sérum de veau foetal décomplémenté: 250 ml
Sang de mouton défibriné : 50 ml
Lécithine : 0,50 g

### Exemple 2B : Milieu solide C15% pour 1000 ml:

### 1- Milieu de Middlebrook 7H10 :

Eau distillée stérile: 750 ml
Sulfate d'ammonium : 0,50 g
Phosphate monopotassique : 1,50 mg
Phosphate disodique : 1,50 g
Citrate de sodium : 0,4 g
Sulfate de magnésium : 0,025 g
Chlorure de calcium : 0,00050 g
Sulfate de zinc : 0,0010 g
Sulfate de cuivre : 0,0010 g
Acide L-glutamique : 0,50 g
Citrate d'ammonium ferrique : 0,04 g
Chlorhydrate de pyridoxine : 0,0010 g
Biotine : 0,000050 g
Vert malachite : 0,0000250 g
Gélose: 15 g

### 2- Facteurs de croissance additionnels:

Chlorure de sodium : 8,50 g
Albumine bovine (fraction V) : 50 g
Dextrose : 20 g
Catalase : 0,030 g
Acide oléïque : 0,60 ml
Glycérol: 0,5%

### 3- Antibiotiques sans activité antimycobactérienne et antifungique :

Polymyxine : 40.000 unités
Amphothéricine B : 4 mg
Acide nalidixique : 16 mg
Triméthoprime : 4 mg
Azlocilline : 4 mg
Vancomycine : 20 mg

### 4- Eléments additionnels de l'invention :

Sérum de veau foetal décomplémenté: 150 ml
Sang de mouton défibriné : 50 ml
Lécithine : 0,50 g

### Exemple 2C : Milieu solide D5% pour 1000 ml:

### 1- Milieu de Middlebrook 7H10 :

Eau distillée stérile: 750 ml
Sulfate d'ammonium : 0,50 g
Phosphate monopotassique : 1,50 mg
Phosphate disodique : 1,50 g
Citrate de sodium : 0,4 g
Sulfate de magnésium : 0,025 g
Chlorure de calcium : 0,00050 g
Sulfate de zinc : 0,0010 g
Sulfate de cuivre : 0,0010 g
Acide L-glutamique : 0,50 g
Citrate d'ammonium ferrique : 0,04 g
Chlorhydrate de pyridoxine : 0,0010 g
Biotine : 0,000050 g
Vert malachite : 0,0000250 g
Gélose: 15 g

### 2- Facteurs de croissance additionnels:

Chlorure de sodium : 8,50 g
Albumine bovine (fraction V) : 50 g
Dextrose : 20 g
Catalase : 0,030 g
Acide oléique : 0,60 ml
Glycérol: 0,5%

### 3- Antibiotiques sans activité antimycobactérienne et antifungique :

Polymyxine : 40.000 unités
Amphothéricine B : 4 mg
Acide nalidixique : 16 mg
Triméthoprime : 4 mg
Azlocilline : 4 mg
Vancomycine : 20 mg

### 4- Eléments additionnels de l'invention :

Sérum de veau foetal décomplémenté: 50 ml
Sang de mouton défibriné : 50 ml
Lécithine : 0,50 g

### EXEMPLE 3 : DECONTAMINATION DES MYCOBACTERIES ET CULTURE EN MILIEU LIQUIDE

Les mycobactéries doivent être isolées et cultivées à partir de prélèvements cliniques normalement contaminés par une flore commensale, par exemple dans le cas des prélèvements d'expectoration qui sont les plus fréquents au laboratoire, ou dans les prélèvements de selles. Plusieurs procédés de décontamination ont été proposés dans la littérature. Il s'agit essentiellement de la décontamination par l'hydroxyde de sodium. Un pré-traitement par un mélange de 2% N-acétyl-L-cystéine mélangé au dithiothreitol permet de réduire la concentration finale d'hydroxyde de sodium entre 1% et 2%. L'expérience des inventeurs concernant l'isolement des mycobactéries du complexe Mycobacterium tuberculosis, notamment à partir des selles, est que ce protocole ne permet pas une décontamination satisfaisante, notamment des selles. Les inventeurs ont donc testé un autre protocole reposant sur l'utilisation de chlorhexidine, décontamination utilisée pour l'isolement des mycobactéries hors complexe Mycobacterium tuberculosis à partir des prélèvements respiratoires chez des patients présentant une mucoviscidose [Ferroni A, Vu-Thien H, Lanotte P, Le Bourgeois M, Sermet-Gaudelus I, Fauroux B, Marchand S, Varaigne F, Berche P, Gaillard JL, Offredo C. Value of the chlorhexidine decontamination method for recovery of nontuberculous mycobacteria from sputum samples of patients with cystic fibrosis. J Clin Microbiol. 2006;44:2237-9]. Cependant, ce procédé est réalisé lors de l'inoculation de milieux solides contenant de l'oeuf (milieu de Lowenstein-Jensen, milieu de Coletsos par exemple), mais n'est pas utilisé dans des milieux de culture liquides. Toutefois, les inventeurs ont découvert que les milieux de culture liquides selon l'invention sont compatibles avec une décontamination à la chlorhexidine pour inactiver les bactéries contaminantes inhibant la croissance des mycobactéries, sans inhiber la croissance des mycobactéries comme décrit ci-après.

Les inventeurs ont, à partir de chlorhexidine digluconate à 20% (Sigma, Illkirch, France), préparé et utilisé de la chlorhexidine digluconate à 1% par dilution extemporanée dans de l'eau distillée stérile. La chlorhexidine diluée se conserve 24 heures.

Le protocole de décontamination était le suivant :
- préparer 5 ml de prélèvement dans un tube Falcon 50 ml
- ajouter 3 volumes (soit 15 ml) de Chlorhexidine digluconate à 1%
- vortexer
- agiter à température ambiante sur agitateur pendant 15 mn
- ajouter 30 ml de tampon phosphate pH 6,8
- agiter par inversion
- centrifuger 20 mn à 3500 tours/mn
- décanter le surnageant
- reprendre le culot dans 500 µl de tampon phosphate pH 6,8
- vortexer
- ensemencer à raison de 200 à 300 µl de cette suspension par flacon.

Ce protocole a été appliqué avec succès à une souche clinique de Mycobacterium gordonae, utilisée comme souche témoin pour l'ensemble du genre Mycobacterium.

### EXEMPLE 4 : ISOLEMENT DES MYCOBACTERIES PAR CULTURE DE PRELEVEMENTS CLINIQUES EN MILIEU LIQUIDE

Dans cet exemple, les inventeurs ont comparé le milieu de leur invention en parallèle au milieu de référence, pour l'isolement des mycobactéries Mycobacterium tuberculosis à partir de prélèvements cliniques montrant la présence de bacilles alcoolo-acido-résistants après coloration de Ziehl-Neelsen. Il est connu dans la littérature médicale que la présence de bacilles alcoolo-acido-résistants témoigne d'un inoculum ≥ 104 mycobactéries / mL de prélèvement [Hobby GL, Holman AP, Iseman MD, Jones JM. Enumeration of tubercle bacilli in sputum of patients with pulmonary tuberculosis. Antimicrob Agents Chemother. 1973;4:94-104].

Dans cet exemple, les inventeurs ont inoculé différents prélèvements cliniques présentant des bacilles alcoolo-acido-résistants évocateurs de mycobactéries décontaminés à la soude, en parallèle dans un bouillon Middlebrook 7H9 additionné d'un mélange d'antibiotiques et d'amphotéricine B (antifongique) commercialisé par Becton-Dickinson sous le sigle PANTA et dans un bouillon de culture de l'exemple 1, comprenant un milieu Middlebrook 7H9-PANTA plus 5% de sang défibriné de mouton, 25% de sérum de veau foetal décomplémenté plus 0,5% de lécithine d'oeuf. Les milieux étaient contenus dans des flacons incubés en parallèle dans un automate BACTEC 9000MB à 37°C avec une détection de consommation d'oxygène comme expliciter ci-dessus. La confirmation de la présence de mycobactéries et leur identification ont été réalisées comme explicité ci-dessus.

Le critère de jugement utilisé est le délai de détection de croissance de mycobactéries par un automate BACTEC 9000MB.

Un prélèvement d'aspiration bronchique a été détecté par culture en 3,22 jours en Middlebrook 7H9 plus sang contre 5 jours en Middlebrook 7H9; un prélèvement d'expectoration a été détecté par culture en 8 jours en Middlebrook 7H9 plus sang et en 13 jours en Middlebrook 7H9 ; un prélèvement de lavage bronchiolo-alvéolaire a été détecté par culture en 4,52 jours en Middlebrook 7H9 plus sang et en 7,34 jours en Middlebrook 7H9. Cet exemple illustre la supériorité du milieu de culture de l'invention pour l'isolement des mycobactéries, en particulier des mycobactéries du complexe Mycobacterium tuberculosis. Ceci constitue un résultat tout à fait important pour le délai du diagnostic de la tuberculose.

### EXEMPLE 5 : IDENTIFICATION DES MYCOBACTERIES PAR SPECTROMETRIE DE MASSE DIRECTEMENT A PARTIR DU MILIEU LIQUIDE

L'identification des mycobactéries à partir d'une culture en milieu liquide repose actuellement sur la coloration de Ziehl, puis une identification moléculaire selon différents procédés publiés dans la littérature. Les limites de ces procédés sont leur délai de plusieurs heures (entre 4 et 24 heures) et leur cout. Les inventeurs ont imaginé mettre à profit l'identification des bactéries par analyse du profil protéique en spectrométrie de masse, pour l'identification rapide de Mycobacterium tuberculosis directement à partir du surnageant de flacon de culture selon une des milieux de l'invention. En effet, l'analyse de profils protéiques par spectrométrie de masse n'a été développée actuellement que pour des souches de mycobactéries isolées et cultivées en milieu solide et non pour des cultures de prélèvements biologiques comprenant des mycobactéries cultivées en milieu solide ou liquide [Hettick JM, Kashon ML, Simpson JP, Siegel PD, Mazurek GH, Weissman DN. Proteomic profiling of intact mycobacteria by matrix-assisted laser desorption/ionization time-of-flight mass spectrometry. Anal Chem. 2004;76:5769-76; Hettick JM, Kashon ML, Slaven JE, Ma Y, Simpson JP, Siegel PD, Mazurek GN, Weissman DN. Discrimination of intact mycobacteria at the strain level: a combined MALDI-TOF MS and biostatistical analysis. Proteomics. 2006;6:6416-25; Pignone M, Greth KM, Cooper J, Emerson D, Tang J. Identification of mycobacteria by matrix-assisted laser desorption ionization-time-of-flight mass spectrometry. J Clin Microbiol. 2006;44:1963-70]. L'utilisation de la spectrométrie de masse, pour l'identification rapide des mycobactéries Mycobacterium tuberculosis directement dans leur prélèvement biologique en culture, s'est heurtée à plusieurs difficultés liées à la nécessité d'inactiver les mycobactéries dès leur prélèvement dans le flacon de culture liquide, sans altérer leur profil protéique. Plusieurs protocoles utilisant par exemple une inactivation des mycobactéries dans le milieu de culture par l'acide tri-fluoro-acétique (TFA) à 2.5% ou par le glutharaldéhyde à 1% n'ont pas permis d'obtenir de spectre discriminant. Finalement, les inventeurs ont obtenu des spectres d'identification en utilisant un protocole d'inactivation par la chaleur comme détaillé ci-dessous :

Le protocole de préparation des bouillons de culture, pour la détection et l'identification des mycobactéries dans les milieux de l'invention par spectrométrie de masse, a été le suivant :
1- dans le laboratoire de niveau de sécurité P3, prélever 5 ml de surnageant à partir du flacon de culture dans cinq tubes stériles (1ml par tube)
2- chauffer 1h à 95°C
3- sortir du laboratoire P3
4- centrifuger à basse vitesse 500 tours/minute 15 min
5- récupérer le surnageant de chacun des cinq tubes dans un nouveau tube stérile
6- centrifuger le surnageant à grande vitesse 14000 tours/minute pendant 10 minutes
7- jeter le surnageant
8- laver le culot avec 1 ml de PBS
9- centrifuger à 14000 tours/minute pendant 10 minutes
10- jeter le surnageant
11- ajouter 5 µL d'acide tri-fluoro-acétique (TFA) à 20%
12- ajouter 5 µl d'acétonitrile à 100%
13- attendre 10min
14- vortexer
15- déposer 1µl du mélange sur la plaque de spectrométrie
16- laisser sécher
17- déposer 1 µl de solution de matrice
18- laisser sécher
19- passer la plaque en spectrométrie de masse.

Pour chaque prélèvement, les inventeurs ont réalisé en parallèle quatre dépôts sur la plaque de spectrométrie de masse. Les dépôts ont été recouverts de 2 µL de solution de matrice, à savoir une solution saturée d'acide alpha-cyano-4-hydroxycinnamique dans de l'acétonitrile à 50%, et de l'acide tri-fluoracétique à 2.5%. La solution de matrice a été cristallisée à température ambiante pendant 5 minutes. Les analyses en spectrométrie de masse ont été réalisées à l'aide d'un spectromètre Autoflex II (Bruker Daltonik) équipé d'un laser azote d'une longueur d'onde de 337-nm. Les spectres ont été enregistrés sur un mode linéaire (délai 170 ns, ion source 1 (IS1) voltage 20 kV; ion source 2 (IS2) voltage 18.5 kV; voltage lentille 7 kV; masse moléculaire entre 2 et 20 kDa). Chaque spectre a été obtenu après 675 tirs sur un mode automatique avec une puissance de laser variable et un temps d'acquisition de 30-60 secondes par dépôt. L'acquisition automatique des données a été faite par le logiciel AutoXecute acquisition control software. Pour chaque échantillon, les quatre spectres obtenus ont été importés dans le logiciel BioTyper version 2.0 software (Bruker Daltonik GmbH) et analysés en utilisant les paramètres par défaut. Les spectres ont été comparés avec une banque locale de spectres des mycobactéries réalisée par les inventeurs. Les espèces de mycobactéries ont été choisies pour être représentatives des groupes/espèces principalement retrouvés en pathologie humaine dans la littérature et dans l'expérience des inventeurs, à savoir Mycobacterium avium ss avium, Mycobaterium avium ss paratuberculosis comme mycobactérie à croissance lente et Mycobacterium smegmalis comme mycobactérie à croissance rapide. La méthode d'analyse incluait des ratios m/z entre 3 et 15 kDa.

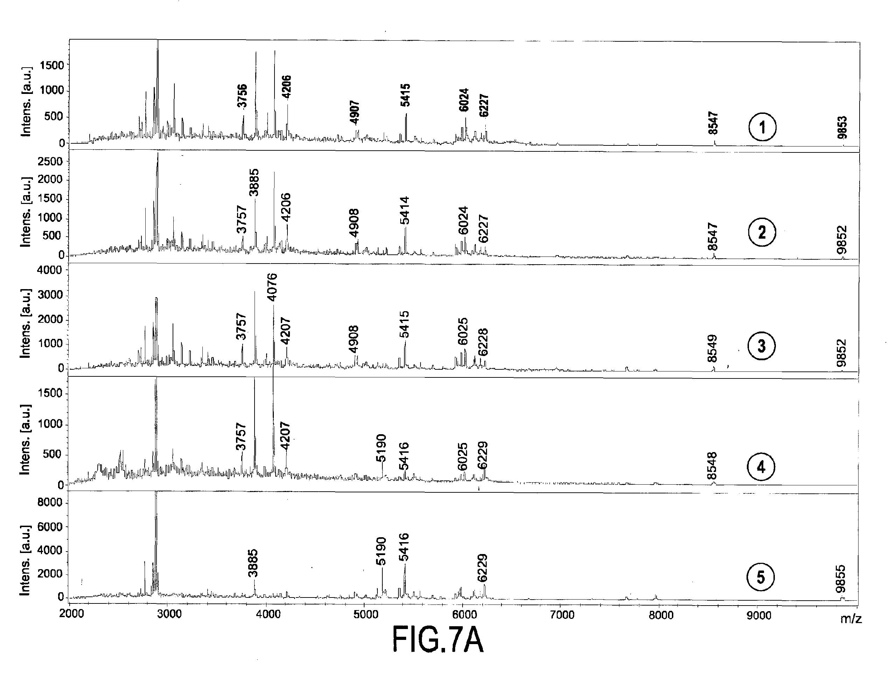

Les figures 7A à 7D représentent des spectres obtenus en spectrométrie de masse à partir de culture en milieu liquide selon l'invention, pour différentes souches de mycobactéries.

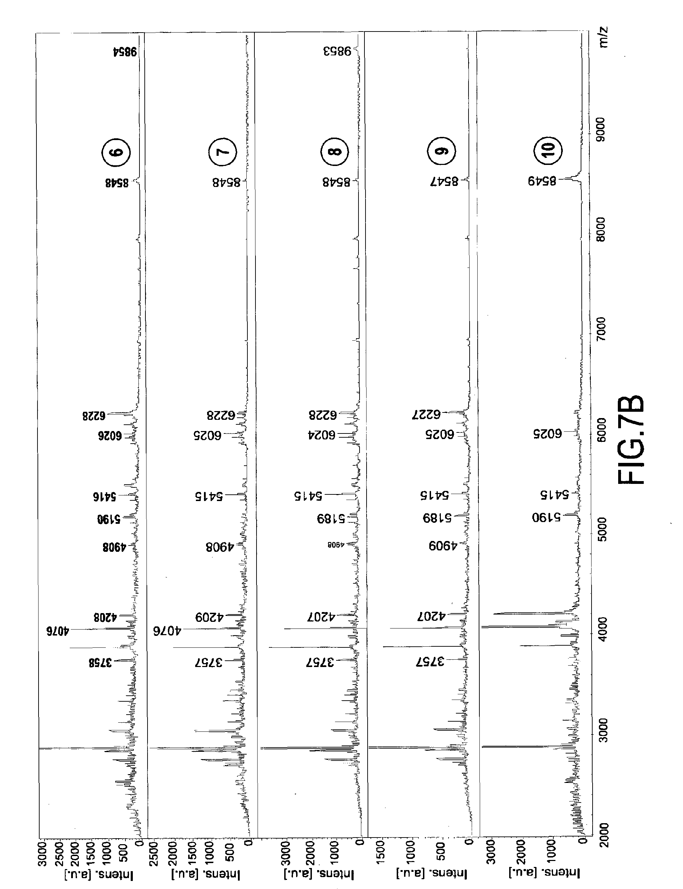

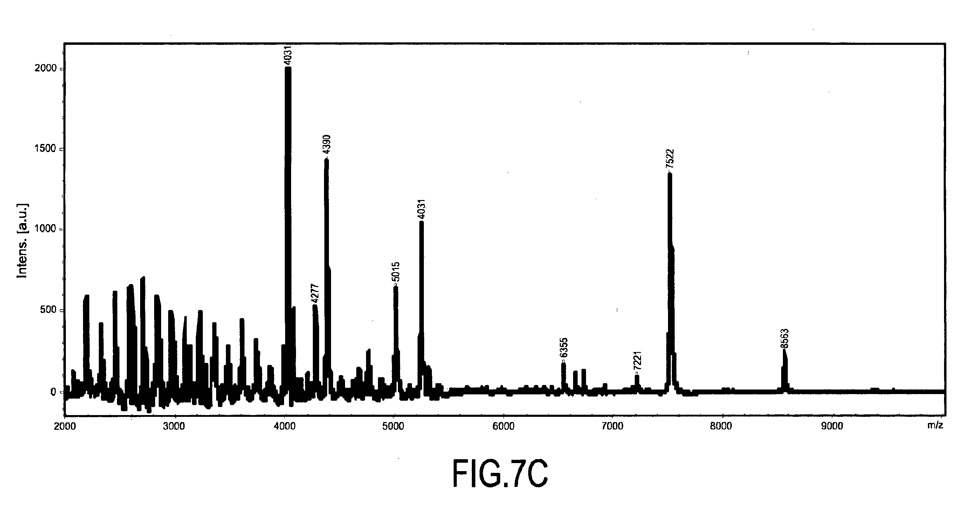

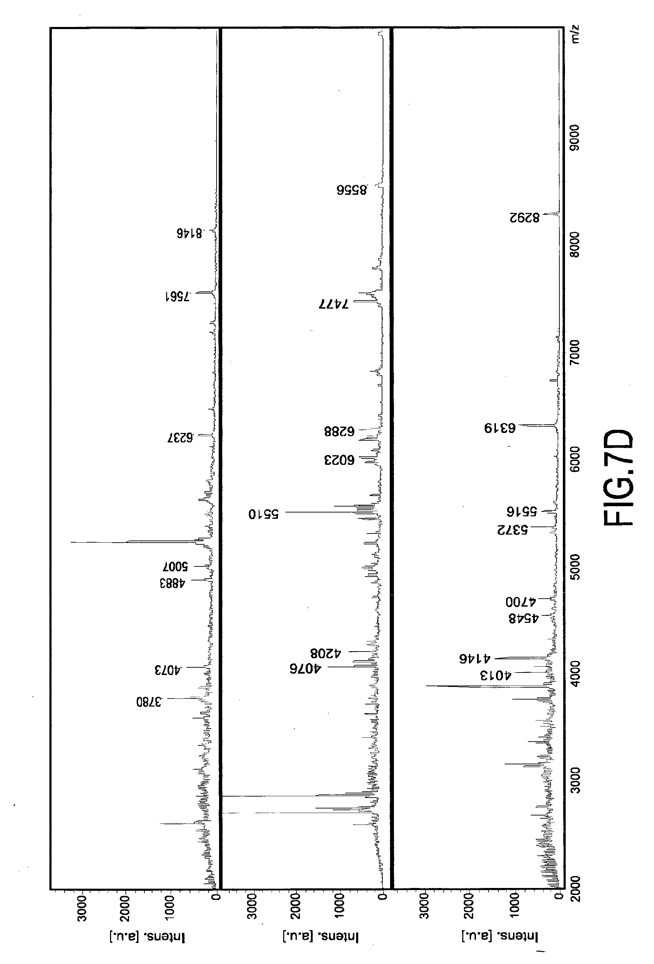

Les figures 7A et 7B représentent des spectres protéiques obtenus par spectrométrie de masse à partir de 10 souches cliniques de Mycobacterium tuberculosis cultivées dans un milieu liquide B25% selon l'invention. La figure 7C représente un spectre témoin négatif obtenu à partir du même milieu liquide B25% non-inoculé et, pour comparaison, la figure 7D représente les spectres obtenus à partir du même milieu liquide B25% selon l'invention inoculé avec Mycobacterium avium ss. avium (11), Mycobacterium avium ss. paratuberculosis (12) et Mycobacterium smegmatis (13).

Les spectres des figures 7A et 7B, pour les différentes dites souches de la même espèce Mycobacterium tuberculosis, présentent un profil extrêmement reproductible d'une souche à l'autre et, en revanche, différent de celui des souches des autres espèces de mycobactéries de la figure 7D, d'une part, et différentes du témoin de la figure 7C, d'autre part.

On observe en comparant les spectres des figures 7A à 7D, que pour Mycobacterium tuberculosis, on obtient spécifiquement des pics correspondant aux quatre protéines ayant les poids moléculaires suivants (en Dalton): 8 548±1; 6 228±1; 6 025±1; 5 415±1.

Le protocole de préparation des bouillons de culture pour la détection et l'identification des mycobactéries dans les milieux solides de l'invention par spectrométrie de masse a été le suivant :
1- prélever 1 ml de surnageant à partir du flacon préalablement agité dans un tube stérile
2- inactiver les mycobactéries par chauffage à 95°C pendant 1 heure
3- faire centrifuger à basse vitesse 500 Tours pendant 15 minutes
4-récupérer le surnageant dans un nouveau tube stérile
5-centrifuger le surnageant à 14000 tours/min pendant 10 minutes
6- jeter le surnageant
7- laver le culot avec 1 ml de solution tampon PBS
8- centrifuger à 14000 Tours pendant 10 minutes
9- jeter le surnageant
10- ajouter 5 µL de TFA à 20%
11- ajouter 5 µl d'acétonitrile à 100%
12- attendre 10 minutes
13- vortexer
14- déposer 1 µl du mélange sur la plaque de spectrométrie
15- laisser sécher
16- déposer 1 µl de solution de matrice
17- laisser sécher 10 minutes à température ambiante
18- passer la plaque en spectrométrie de masse.

Toutes les manipulations doivent être effectuées en tenue complète (charlotte, masque, surblouse et gants) sous la hotte.

### EXEMPLE 6 : DETERMINATION PHENOTYPIQUE DE LA SENSIBILITE DE M. TUBERCULOSIS AUX ANTIBIOTIQUES

### a/- Méthodes :

Les inventeurs ont testé six souches cliniques de l'espèce Mycobacterium tuberculosis isolées de prélèvements respiratoires au cours du diagnostic de routine dans leur laboratoire de microbiologie clinique. Ces six souches ont été identifiées de l'espèce Mycobacterium tuberculosis par leur morphologie caractéristique en cordons après coloration de Ziehl, puis par amplification PCR et séquençage de spacers intergéniques [Djelouadji Z et al. A Single-Step Sequencing Method for the Identification of Mycobacterium tuberculosis Complex Species. PLoS Negl Trop Dis. 2008 Jun 18;2(6):e253]. Ces six souches ont été testées sensibles aux antibiotiques antituberculeux de première ligne en utilisant un test phénotypique de référence en milieu solide ne testant que les concentrations dites critiques pour chacun des quatre antibiotiques testés : isoniazide, 0,2 µg/ml ; rifampicine, 1 µg/ml ; ethambutol, 5 µg/ml et streptomycine, 5 µg/ml. [Woods GL, Warren NG, Inderlind CB. Susceptibility test methods : Mycobacteria, Nocardia, and other Actinomycetes in: Manual of Clinical Microbiology, 9ème édition (Nurray PR, Baren EJ, Jorgensen JH, Bry NL, Pfaller MA. American Society for Microbiology, 2007, page 1223-1247]. On a testé en parallèle une souche clinique résistante aux antituberculeux (concentration minimale inhibitrice de la rifampicine > 1 µg/ml) comme déterminé par la méthode de référence en milieu solide [Woods GL, Warren NG, Inderlind CB. Susceptibility test methods : Mycobacteria, Nocardia, and over actinomycetes in: Manual of Clinical Microbiology, 9ème édition (Nurray PR, Baren EJ, Jorgensen JH, Bry NL, Pfaller MA. American Society for Microbiology, 2007, page 1223-1247). Ces souches ont été mises en suspension dans du tampon phosphate stérile à la concentration de 10⁶ colonies/ml. Cette suspension a servi à inonder en parallèle une boite de Pétri stérile contenant le milieu standard de référence : milieu Middlebrook 7H10 complémenté par un mélange d'acide oléique, albumine, dextrose, et catalase (OADC) et une boîte de Pétri stérile contenant le milieu solide 15% selon l'invention de l'exemple 2B ci-dessus.

Les inventeurs ont utilisé le système appelé E-test^{®} (Biomérieux, France) pour déterminer la sensibilité des souches aux différents antibiotiques. Ce système est composé d'une bandelette en papier imprégnée d'antibiotique (isoniazide ou éthambutol) selon un gradient de concentration continu au long de la bandelette. Ce dispositif permet une lecture à l'oeil nu de la concentration à partir de laquelle il n'est plus observé de croissance de Mycobacterium tuberculosis définissant ainsi de la concentration minimum inhibitrice de l'antibiotique (isoniazide ou éthambutol) vis-à-vis de la souche de Mycobacterium tuberculosis qui est testée. Les boîtes de Pétri ont été incubées à 37°C sous une atmosphère contenant 5% de CO₂ pendant deux semaines avec une observation quotidienne à partir du sixième jour d'incubation. Les colonies ont été confirmées comme étant Mycobacterium tuberculosis sur la base de leur aspect et après coloration de Ziehl montrant des cordons de bacilles alcoolo-acido-résistants. Il se forme un disque d'inhibition de la croissance bactérienne autour de la bandelette dans les zones contenant une concentration d'antibiotique supérieure à la concentration minimum inhibitrice, étant entendu que celle-ci est déterminée par l'intersection du disque d'inhibition avec la bandelette.

### b/- Résultats :

Les inventeurs ont observé que :
- (1) les colonies de Mycobacterium tuberculosis apparaissaient après 7 jours d'incubation sur le milieu selon l'invention et après 11 jours d'incubation sur le milieu de référence,
- (2) la lecture du E-test est possible dans ces conditions après 7 jours d'incubation sur le milieu selon l'invention car il y a un nombre suffisant de colonies permettant la lecture et un très bon contraste entre les colonies qui apparaissent grises sur le fond rouge du milieu selon l'invention; alors que la lecture est seulement partielle et délicate après 11 jours d'incubation sur le milieu de référence, du fait de la croissance incomplète des colonies sur le milieu de référence et d'un mauvais contraste entre la couleur de la colonie (translucide) et le milieu de référence,
- (3) les résultats des E-tests isoniazide et éthambutol obtenus sur le milieu selon l'invention sont similaires à ceux obtenus au préalable par la méthode de référence,
- (4) les résultats des E-tests isoniazide et éthambutol obtenus sur le milieu selon l'invention sont plus précis que ceux des tests de référence, en permettant la détermination d'une véritable concentration minimale inhibitrice.

Cette expérience démontre que, par utilisation d'un milieu solide selon l'invention, la réalisation de tests phénotypiques de sensibilité de Mycobacterium tuberculosis aux antituberculeux selon la méthode E-test, est plus rapide et plus facile que la réalisation de E-test sur un milieu solide de référence.

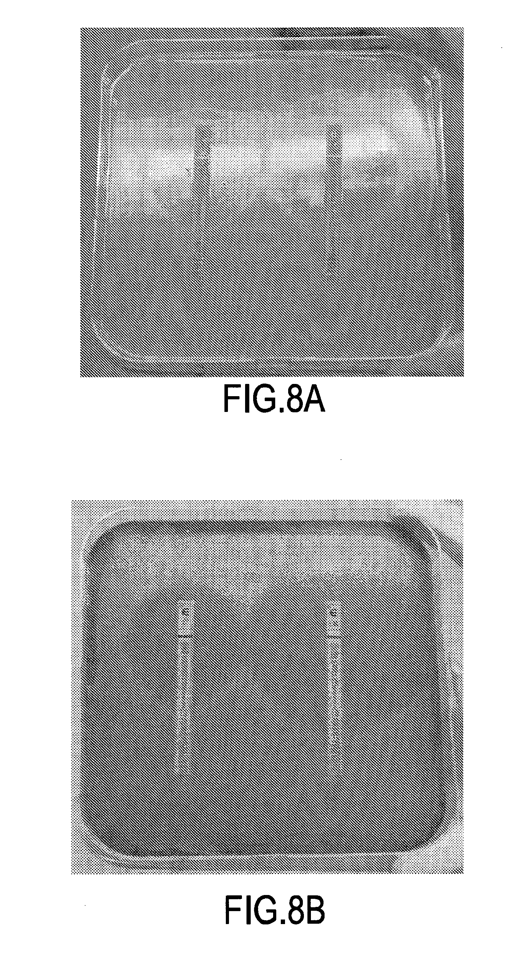

Sur les figures 8A et 8B, sont représentées des photographies d'une boîte de Pétri contenant le milieu de référence (figure 8A) et le milieu selon l'invention (figure 8B), incubée après inoculation d'une même souche de l'espèce Mycobacterium tuberculosis, montrant la présence de colonies de Mycobacterium tuberculosis facilement visibles après 7 jours sur le milieu selon l'invention (figure 8B) et difficilement visibles sur le milieu de référence après 11 jours (figure 8A), permettant une lecture facile de la concentration inhibitrice de isoniazide (IZ) sur une première bandelette et éthambutol (ET) sur une deuxième bandelette.

Sur les figures 8A et 8B, on voit, autour des bandelettes, des zones d'inhibition de la croissance bactérienne en forme de disque ovoïde autour des parties de la bandelette contenant une concentration inhibitrice d'antibiotique, de sorte que l'intersection de la limite inférieure desdites zones inhibitrices avec les bandelettes permet de lire sur la bandelette graduée la concentration inhibitrice minimale de l'antibiotique.

## Revendications

1. Milieu de culture de mycobactéries, comprenant des facteurs de croissance de mycobactéries et, de préférence, des antibiotiques sans activité à l'égard des mycobactéries, **caractérisé en ce qu'**il comprend les composants additionnels suivants :
- de la lécithine dans une proportion pondérale d'au moins 0,1%, et
- du sang défibriné dans une proportion en volume d'au moins 2,5%, et
- du sérum de veau foetal décomplémenté dans une proportion en volume d'au moins 2,5%.

2. Milieu de culture de mycobactéries selon la revendication 1, **caractérisé en ce que** :
- la lécithine est comprise dans une proportion pondérale de 0,1 à 5%, de préférence 0,5 à 1%, et
- le sang est compris dans une proportion en volume de 2,5 à 15%, de préférence de 5 à 10%, et
- le sérum de veau foetal décomplémenté est compris dans une proportion en volume de 2,5 à 25%, de préférence de 10 à 20%.

3. Milieu de culture de mycobactéries selon la revendication 2, **caractérisé en ce que** :
- la lécithine est de la lécithine de jaune d'oeuf, et
- le sang est du sang défibriné de lapin ou, de préférence, de mouton.

4. Milieu de culture de mycobactéries selon l'une des revendications 1 à 3, **caractérisé en ce qu'**il comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine.

5. Milieu de culture de mycobactéries selon l'une des revendications 1 à 4, **caractérisé en ce que** ledit milieu de culture est un milieu de culture liquide.

6. Milieu de culture de mycobactéries selon la revendication 5, **caractérisé en ce qu'**il comprend un milieu de culture de base qui est un milieu liquide Middlebrook de référence 7H9 et des facteurs de croissance de mycobactéries additionnels suivants : hydrolysat de caséine, glycérol, polysorbate, hemin, acide lactique, biotine, stéarate de polyoxyéthylène, sérum albumine bovine, dextrose et supplément H.

7. Milieu de culture de mycobactéries selon l'une des revendications 1 à 4, **caractérisé en ce que** ledit milieu de culture est un milieu de culture solide contenant un produit gélifiant choisi de préférence parmi les gélose et agar, de préférence dans une proportion pondérale de 0.5 à 5%, de préférence encore de 1 à 2%.

8. Milieu de culture de mycobactéries selon la revendication 7, **caractérisé en ce qu'**il s'agit d'un milieu solide de type Middlebrook de référence 7H10, additionné de dit produit gélifiant et des facteurs de croissance de mycobactéries additionnels suivants : acide oléique, albumine bovine, de préférence la fraction V de l'albumine bovine, dextrose, catalase et glycérol.

9. Procédé de culture d'une mycobactérie à l'aide d'un milieu de culture de mycobactéries selon l'une des revendications 1 à 8, **caractérisé en ce que** l'on chauffe un échantillon contenant une dite mycobactérie, dans un dit milieu de culture de mycobactéries, à une température de 30 à 37°C, appropriée pour la culture de l'espèce de mycobactérie contenue dans l'échantillon.

10. Procédé de culture d'une mycobactérie selon la revendication 9, **caractérisé en ce que** l'on cultive un échantillon contenant une bactérie du complexe *Mycobacterium tuberculosis* à une température de 37°C dans un dit milieu de culture de mycobactéries.

11. Procédé de culture d'une mycobactérie selon la revendication 9 ou 10, **caractérisé en ce que** l'on réalise les étapes suivantes:
- on effectue la culture d'un échantillon de prélèvement biologique pouvant contenir des mycobactéries jusqu'à ce qu'une croissance de bactéries soit détectable, et
- on identifie que la bactérie détectée est une bactérie du genre mycobactérie par un test de coloration, et
- le cas échéant, on identifie l'espèce de ladite mycobactérie par des moyens d'analyse moléculaire, de préférence par spectrométrie de masse.

12. Procédé de culture d'une mycobactérie selon l'une des revendications 9 à 11, dans lequel on réalise une culture d'un échantillon de prélèvement biologique pouvant contenir une mycobactérie et des bactéries contaminantes dont la croissance peut inhiber la croissance des mycobactéries, **caractérisé en ce que** :
1/ on réalise une étape préalable de décontamination initiale dudit échantillon dans ledit milieu de culture avec de la chlorhexidine pendant une période limitée, afin de limiter l'action de la chlorhexidine à une activité contre les bactéries autres que mycobactéries, de préférence environ 15 minutes de traitement dans une solution de chlorhexidine à 1% sous agitation, et
2/ on élimine la chlorhexidine en lavant l'échantillon ainsi traité de l'étape 1/ avec un tampon neutre, et on centrifuge et récupère le culot bactérien contenant les bactéries contaminantes ainsi inactivées et les mycobactéries non inactivées, que l'on inocule sur un dit milieu de culture de mycobactéries.

13. Procédé de culture d'une mycobactérie selon l'une des revendications 9 à 12, **caractérisé en ce que** l'on réalise une culture d'un échantillon de prélèvement biologique, ledit prélèvement biologique étant effectué à partir de selles.

14. Procédé de culture d'une mycobactérie selon l'une des revendications 9 à 13, **caractérisé en ce que** ledit milieu de culture est un dit milieu de culture liquide de mycobactéries et on détecte la croissance de dites mycobactéries par analyse périodique de la concentration en oxygène au sein de l'enceinte de culture.

15. Procédé de culture et d'identification d'une mycobactérie dans un prélèvement d'échantillon biologique selon l'une des revendications 11 à 14, **caractérisé en ce que** l'on effectue la culture dudit échantillon de prélèvement biologique dans un dit milieu de culture de mycobactéries liquide, et on identifie l'espèce de bactéries du genre mycobactérie par des moyens d'analyse moléculaire consistant dans une analyse du profil protéique obtenu par spectrométrie de masse, en le comparant à une série de spectres de profil protéique obtenus avec des échantillons de souche de mycobactéries de référence de différentes espèces cultivées dans les mêmes conditions de culture, et, pour la réalisation de l'analyse de spectrométrie de masse, on analyse un culot bactérien obtenu par double centrifugation, directement à partir du bouillon de culture selon les étapes suivantes :
a- on inactive les bactéries d'un échantillon de prélèvement biologique cultivé, dans lequel on a détecté la croissance de bactéries, par chauffage à plus de 90°C, de préférence à 95°C, pendant 1 heure, de préférence sous agitation,
b- on réalise une première centrifugation à basse vitesse, de préférence à 500 tr/mn, du surnageant d'un dit échantillon de prélèvement biologique cultivé, dans lequel on a détecté la croissance de bactéries, cette première centrifugation étant réalisée jusqu'à sédimenter les globules rouges du sang contenu dans ledit échantillon, et
c- on récupère le surnageant de la première centrifugation de l'étape b-, et on réalise une deuxième centrifugation à grande vitesse, de préférence au moins 10 000 tr/mn, de préférence environ 14 000 tr/mn, cette deuxième centrifugation étant réalisée jusqu'à obtenir sédimentation d'un culot bactérien, et
d- de préférence, on lave ledit culot bactérien de l'étape c- avec un tampon neutre, tel que du PBS, et on réalise un traitement chimique, de préférence avec un mélange d'acétonitrile et d'acide trifluoroacétique, pour séparer les protéines de la bactérie et les rendre analysable par spectrométrie de masse, et
e- on récupère le culot protéique bactérien de l'étape d- que l'on dépose sur une plaque de spectrométrie de masse.

16. Procédé de culture d'une mycobactérie selon l'une des revendications 9 à 13, dans laquelle on met en oeuvre un milieu de culture solide, **caractérisé en ce que** l'on réalise les étapes dans lesquelles :
i/- on réalise une culture d'une dite mycobactérie, de préférence du complexe Mycobacterium tuberculosis, sur dit un milieu de culture solide en présence d'au moins un antibiotique donné, à différentes concentrations connues, et
ii/- on détermine la plus petite concentration d'antibiotique, de préférence choisi parmi la rifampicine, isoniazide, éthambutol, pyrazinamide et la streptomycine, qui inhibe toute croissance visible de ladite bactérie.

## Patentansprüche

1. Medium zur Kultivierung von Mykobakterien, umfassend Wachstumsfaktoren für Mykobakterien sowie vorzugsweise Antibiotika ohne Aktivität hinsichtlich der Mykobakterien, **dadurch gekennzeichnet, dass** es die folgenden zusätzlichen Bestandteile umfasst:
- Lecithin in einem Gewichtsanteil von wenigstens 0,1 % und
- defibriniertes Blut in einem Volumenanteil von wenigstens 2,5 % und
- dekomplementiertes fetales Kälberserum in einem Volumenanteil von wenigstens 2,5 %.

2. Medium zur Kultivierung von Mykobakterien nach Anspruch 1, **dadurch gekennzeichnet, dass**:
- das Lecithin in einem Gewichtsanteil von 0,1 bis 5 %, vorzugsweise von 0,5 bis 1 % enthalten ist und
- das Blut in einem Volumenanteil von 2,5 bis 15 %, vorzugsweise von 5 bis 10 % enthalten ist und
- das dekomplementierte fetale Kälberserum in einem Volumenanteil von 2,5 bis 25 %, vorzugsweise von 10 bis 20 % enthalten ist.

3. Medium zur Kultivierung von Mykobakterien nach Anspruch 2, **dadurch gekennzeichnet, dass**:
- das Lecithin Lecithin aus Eigelb ist und
- das Blut defibriniertes Blut vom Kaninchen oder, vorzugsweise, vom Schaf ist.

4. Medium zur Kultivierung von Mykobakterien nach einem der Ansprüche 1 bis 3, **dadurch gekennzeichnet, dass** es ein Basis-Mykobakterienkulturmedium umfasst, das neben destilliertem Wasser die folgenden Bestandteile umfasst: Ammoniumsulfat, Magnesiumsulfat, Kupfersulfat, Zinksulfat, Natriumcitrat, Ammoniumeisen(III)-citrat, Natriumchlorid, Calciumchlorid, Monokaliumphosphat, Dinatriumphosphat, L-Glutaminsäure, Biotin und Pyridoxin.

5. Medium zur Kultivierung von Mykobakterien nach einem der Ansprüche 1 bis 4, **dadurch gekennzeichnet, dass** das Kulturmedium ein flüssiges Kulturmedium ist.

6. Medium zur Kultivierung von Mykobakterien nach Anspruch 5, **dadurch gekennzeichnet, dass** es ein Basiskulturmedium, welches ein flüssiges Middlebrook-Medium 7H9 ist, sowie folgende zusätzliche Mykobakterien-Wachstumsfaktoren umfasst: Caseinhydrolysat, Glycerin, Polysorbat, Hemin, Milchsäure, Biotin, Polyoxyethylenstearat, bovines Serumalbumin, Dextrose und Ergänzung H.

7. Medium zur Kultivierung von Mykobakterien nach einem der Ansprüche 1 bis 4, **dadurch gekennzeichnet, dass** das Kulturmedium ein festes Kulturmedium ist, das ein Geliermittel enthält, welches vorzugsweise aus Gelose und Agar, vorzugsweise in einem Gewichtsanteil von 0,5 bis 5 %, weiterhin vorzugsweise von 1 bis 2 %, ausgewählt ist.

8. Medium zur Kultivierung von Mykobakterien nach Anspruch 7, **dadurch gekennzeichnet, dass** es sich um ein festes Medium vom Typ Middlebrook 7H10 handelt, dem Geliermittel sowie folgende zusätzliche Mykobakterien-Wachstumsfaktoren zugegeben sind: Ölsäure, Rinderalbumin, vorzugsweise die Faktion V von Rinderalbumin, Dextrose, Katalase und Glycerin.

9. Verfahren zur Kultivierung eines Mykobakteriums mit Hilfe eines Mediums zur Kultivierung von Mykobakterien nach einem der Ansprüche 1 bis 8, **dadurch gekennzeichnet, dass** eine ein Mykobakterium enthaltende Probe in einem Mykobakterienkulturmedium auf eine Temperatur von 30 bis 37 °C, die für die Kultivierung der in der Probe enthaltenen Mykobakterienspezies geeignet ist, erhitzt wird.

10. Verfahren zur Kultivierung eines Mykobakteriums nach Anspruch 9, **dadurch gekennzeichnet, dass** eine Probe, die ein Bakterium des *Mycobacterium tuberculosis-*Komplexes enthält, bei einer Temperatur von 37 °C in einem Mykobakterienkulturmedium kultiviert wird.

11. Verfahren zur Kultivierung eines Mykobakteriums nach Anspruch 9 oder 10, **dadurch gekennzeichnet, dass** die folgenden Schritte durchgeführt werden:
- es wird die Kultivierung einer biologischen Probe, welche Mykobakterien enthalten kann, vollzogen, bis ein Bakterienwachstum nachweisbar ist, und
- durch einen Färbungstest wird festgestellt, dass das nachgewiesene Bakterium ein Bakterium der Gattung Mykobakterium ist, und
- gegebenenfalls wird die Spezies des Mykobakteriums durch Mittel zur molekularen Analyse, vorzugsweise durch Massenspektrometrie festgestellt.

12. Verfahren zur Kultivierung eines Mykobakteriums nach einem der Ansprüche 9 bis 11, bei dem ein Kultivieren einer biologischen Probe, welche ein Mykobakterium und kontaminierende Bakterien, deren Wachstum das Wachstum der Mykobakterien hemmen kann, enthalten kann, durchgeführt wird, **dadurch gekennzeichnet, dass**:
1/ ein vorhergehender Schritt zur Anfangsdekontamination der Probe in dem Kulturmedium mit Chlorhexidin über einen begrenzten Zeitraum durchgeführt wird, um die Wirkung des Chlorhexidins auf eine Aktivität gegen die anderen Bakterien als die Mykobakterien zu begrenzen, vorzugsweise etwa 15 Minuten Behandlung in einer 1 %igen Chlorhexidin-Lösung unter Rühren, und
2/ das Chlorhexidin dadurch entfernt wird, dass die auf diese Weise behandelte Probe des Schrittes 1/ mit einem neutralen Puffer gewaschen wird, und der bakterielle Rückstand, welcher die auf diese Weise deaktivierten kontaminierenden Bakterien und die nicht deaktivierten Mykobakterien enthält, zentrifugiert und abgenommen wird, der in ein Medium zur Kultivierung von Mykobakterien geimpft wird.

13. Verfahren zur Kultivierung eines Mykobakteriums nach einem der Ansprüche 9 bis 12, **dadurch gekennzeichnet, dass** ein Kultivieren einer biologischen Probe vollzogen wird, wobei die biologische Probenentnahme an Stuhl vorgenommen wird.

14. Verfahren zur Kultivierung eines Mykobakteriums nach einem der Ansprüche 9 bis 13, **dadurch gekennzeichnet, dass** das Kulturmedium ein flüssiges Mykobakterienkulturmedium ist und das Wachstum von Mykobakterien durch periodische Analyse der Sauerstoffkonzentration innerhalb des Kulturraumes nachgewiesen wird.

15. Verfahren zur Kultivierung und zur Identifikation eines Mykobakteriums in einer biologischen Probe nach einem der Ansprüche 11 bis 14, **dadurch gekennzeichnet, dass** die Kultivierung der biologischen Probe in einem flüssigen Mykobakterienkulturmedium durchgeführt wird und die Spezies von Bakterien der Gattung Mykobakterium durch Mittel zur molekularen Analyse identifiziert wird, bestehend in einer Analyse des durch Massenspektrometrie erhaltenen Proteinprofils, durch dessen Vergleich mit einer Reihe von Proteinprofil-Spektren, die mit Proben eines Referenzmykobakterienstammes unterschiedlicher, unter den gleichen Kulturbedingungen kultivierter Spezies erhalten werden, und für die Durchführung der Massenspektrometrieanalyse ein durch zweifaches Zentrifugieren erhaltener Bakterienrückstand direkt anhand der Nährbouillon nach folgenden Schritten analysiert wird:
a- die Bakterien einer kultivierten biologischen Probe, in der das Wachstum von Bakterien nachgewiesen wurde, werden durch Erhitzen auf mehr als 90 °C, vorzugsweise auf 95 °C, für 1 Stunde, vorzugsweise unter Rühren deaktiviert,
b- es wird ein erstes Zentrifugieren mit niedriger Geschwindigkeit, vorzugsweise mit 500 U/Min., des Überstands einer kultivierten biologischen Probe, in der das Wachstum von Bakterien nachgewiesen wurde, durchgeführt, wobei dieses erste Zentrifugieren bis zur Senkung der roten Blutkörperchen des in der Probe enthaltenen Blutes durchgeführt wird, und
c- der Überstand des ersten Zentrifugierens des Schrittes b- wird abgenommen, und es wird ein zweites Zentrifugieren mit hoher Geschwindigkeit, vorzugsweise wenigstens 10.000 U/Min., vorzugsweise etwa 14.000 U/Min. durchgeführt, wobei dieses zweite Zentrifugieren durchgeführt wird, bis das Sedimentieren eines Bakterienrückstandes erreicht wird, und
d- vorzugsweise wird der Bakterienrückstand des Schrittes c- mit einem neutralen Puffer, wie PBS gewaschen, und es wird eine chemische Behandlung, vorzugsweise mit einem Gemisch aus Acetonitril und Trifluoressigsäure durchgeführt, um die Proteine von dem Bakterium abzusondern und sie durch Massenspektrometrie analysierbar zu machen, und
e- der Bakterienproteinrückstand des Schrittes d- wird abgenommen, der auf eine Massenspektrometrieplatte aufgebracht wird.

16. Verfahren zur Kultivierung eines Mykobakteriums nach einem der Ansprüche 9 bis 13, bei dem ein festes Kulturmedium verwendet wird, **dadurch gekennzeichnet, dass** die Schritte durchgeführt werden, bei denen:
i/- ein Kultivieren eines Mykobakteriums, vorzugsweise des *Mycobacterium tuberculosis*-Komplexes, auf einem festen Kulturmedium in Gegenwart wenigstens eines gegebenen Antibiotikums, in bekannten unterschiedlichen Konzentrationen durchgeführt wird und
ii/- die geringste Konzentration an Antibiotikum, das vorzugsweise aus Rifampicin, Isoniazid, Ethambutol, Pyrazinamid und Streptomycin ausgewählt ist, bestimmt wird, die jedes sichtbare Wachstum des Bakteriums hemmt.

## Claims

1. A culture medium for mycobacteria, comprising growth factors for mycobacteria and, preferably, antibiotics without activity against mycobacteria, the culture medium being **characterized in that** it comprises the following additional components:
- lecithin in a proportion by weight of at least 0.1%;
- defibrinated blood in a proportion by volume of at least 2.5%; and
- decomplemented fetal calf serum in a proportion by volume of at least 2.5%.

2. A culture medium for mycobacteria according to claim 1, **characterized in that**:
- lecithin is contained in a proportion by weight from 0.1% to 5%, preferably from 0.5% to 1%;
- the blood is contained in a proportion by volume from 2.5% to 15%, preferably from 5 % to 10%; and
- decomplemented fetal calf serum is contained in a proportion by volume from 2.5% to 25%, preferably from 10% to 20%.

3. A culture medium for mycobacteria according to claim 2, **characterized in that**:
- the lecithin is egg yolk lecithin; and
- the blood is defibrinated rabbit or, preferably, sheep blood.

4. A culture medium for mycobacteria according to any one of claims 1 to 3, **characterized in that** it comprises a basic culture medium for mycobacteria comprising, besides distilled water, the following components: ammonium sulfate, magnesium sulfate, copper sulfate, zinc sulfate, sodium citrate, ferric ammonium citrate, sodium chloride, calcium chloride, monopotassium phosphate, disodium phosphate, L-glutamic acid, biotin and pyridoxine.

5. A culture medium for mycobacteria according to any one of claims 1 to 4, **characterized in that** said culture medium is a liquid culture medium.

6. A culture medium for mycobacteria according to claim 5, **characterized in that** it comprises a basic culture medium which is a Middlebrook liquid medium of reference 7H9 and the following additional growth factors for mycobacteria: casein hydrolyzate, glycerol, polysorbate, hemin, lactic acid, biotin, polyoxyethylene stearate, bovine serum albumin, dextrose and supplement H.

7. A culture medium for mycobacteria according to any one of claims 1 to 4, **characterized in that** said culture medium is a solid culture medium containing a gelling agent preferably selected from the agar gels, preferably in a proportion by weight from 0.5% to 5%, more preferably from 1% to 2%.

8. A culture medium for mycobacteria according to claim 7, **characterized in that** it is a solid medium of the Middlebrook type of reference 7H10, with addition of said gelling agent and the following additional growth factors for mycobacteria: oleic acid, bovine albumin, preferably fraction V of bovine albumin, dextrose, catalase and glycerol.

9. A method of culture of a mycobacterium by means of a culture medium for mycobacteria according to any one of claims 1 to 8, **characterized in that** a sample containing a said mycobacterium is heated in a said culture medium for mycobacteria, at a temperature from 30°C to 37°C, suitable for culture of the species of mycobacterium contained in the sample.

10. A method of culture of a mycobacterium according to claim 9, **characterized in that** a sample containing a bacterium of the *Mycobacterium tuberculosis* complex is cultured at a temperature of 37°C in a said culture medium for mycobacteria.

11. A method of culture of a mycobacterium according to claim 9 or claim 10, **characterized in that** the following stages are carried out:
- culture of a biological sample that can contain mycobacteria until growth of bacteria is detectable;
- identification that the bacterium detected is a bacterium of the genus mycobacterium by a staining test; and
- if necessary, identification of the species of said mycobacterium by means for molecular analysis, preferably by mass spectrometry.

12. A method of culture of a mycobacterium according to any one of claims 9 to 11, in which a culture of a biological sample that can contain a mycobacterium and contaminating bacteria whose growth can inhibit the growth of mycobacteria is carried out, the method of culture being **characterized in that**:
1/ a preliminary stage of initial decontamination of said sample in said culture medium with chlorhexidine is carried out for a limited period, in order to limit the action of the chlorhexidine to activity against bacteria other than mycobacteria, preferably about 15 minutes of treatment in a 1% chlorhexidine solution with stirring; and
2/ the chlorhexidine is removed by washing the sample thus treated from stage 1/ with a neutral buffer, followed by centrifugation and recovery of the bacterial pellet containing the contaminating bacteria thus inactivated and the mycobacteria that have not been inactivated, which is inoculated on a said culture medium for mycobacteria.

13. A method of culture of a mycobacterium according to any one of claims 9 to 12, **characterized in that** culture of a biological sample is carried out, said biological sample being obtained from stool samples.

14. A method of culture of a mycobacterium according to any one of claims 9 to 13, **characterized in that** said culture medium is a liquid said culture medium for mycobacteria and the growth of said mycobacteria is detected by periodical analysis of the oxygen concentration within the culture vessel.

15. A method of culture and of identification of a mycobacterium in a biological sample according to any one of claims 11 to 14, **characterized in that** culture of said biological sample is carried out in a said liquid culture medium for mycobacteria, and the species of bacteria of the genus mycobacterium is identified by molecular analysis means consisting of analysis of the protein profile obtained by mass spectrometry, by comparing it with a series of protein profile spectra obtained with samples of reference strain of mycobacteria of various species cultured in the same culture conditions, and, for carrying out the mass spectrometry analysis, analysis is performed on a bacterial pellet obtained by double centrifugation, directly from the culture broth according to the following stages:
a- the bacteria of a cultured biological sample, in which growth of bacteria has been detected, are inactivated by heating at above 90°C, preferably to 95°C for 1 hour, preferably with stirring;
b- a first centrifugation is carried out at low speed, preferably at 500 round/min, of the supernatant of a said cultured biological sample, in which growth of bacteria has been detected, said first centrifugation being carried out until there is sedimentation of the red blood cells from the blood contained in said sample;
c- the supernatant is recovered from the first centrifugation of stage b-, and a second centrifugation is carried out at high speed, preferably at least 10,000 round/min, preferably about 14,000 round/min, this second centrifugation being carried out to obtain sedimentation of a bacterial pellet;
d- preferably, said bacterial pellet from stage c- is washed with a neutral buffer, such as PBS, and a chemical treatment is carried out, preferably with a mixture of acetonitrile and trifluoroacetic acid, to separate the proteins from the bacterium so that they can be analyzed by mass spectrometry; and
e- the bacterial protein pellet from stage d- is recovered and is deposited on a mass spectrometry plate.

16. A method of culture of a mycobacterium according to any one of claims 9 to 13, employing a solid culture medium, the method of culture being **characterized in that** stages are carried out in which:
i/- a said mycobacterium is cultured, preferably of the Mycobacterium tuberculosis complex, on said solid culture medium in the presence of at least one given antibiotic, at different known concentrations; and
ii/- the lowest concentration of antibiotic, preferably selected from rifampicin, isoniazid, ethambutol, pyrazinamide and streptomycin, that inhibits all visible growth of said bacterium, is determined.
